# EUROPÄISCHE PATENTSCHRIFT

(11) **EP 1 227 786 B1**
(45) Veröffentlichungstag und Bekanntmachung des Hinweises auf die Patenterteilung: **24.08.2005**
(21) Anmeldenummer: 01949088.7
(22) Anmeldetag: 25.01.2001
(51) Int. Cl.: A61K 7/13

(54) **MITTEL ZUR FÄRBUNG VON FASERN ENTHALTEND EIN INDOLIN-/INDOLIUMDERIVAT**
AGENT FOR DYEING FIBRES COMPRISING AN INDOLINE/INDOLIUM DERIVATIVE
AGENT COLORANT POUR FIBRES, CONTENANT UN DERIVE D'INDOLINE/D'INDOLIUM

(30) Priorität: 22.02.2000 DE 10007948
(43) Veröffentlichungstag der Anmeldung: 07.08.2002
(73) Patentinhaber: Wella Aktiengesellschaft, 64274 Darmstadt (DE)
(72) Erfinder: SAUTER, Guido, CH-3174 Thörishaus (CH); BRAUN, Hans-Jürgen, CH-3182 Überstorf (CH); REICHLIN, Nadia, CH-1482 Cugy (CH)
(86) Internationale Anmeldenummer: PCT/EP2001/000821
(87) Internationale Veröffentlichungsnummer: WO 2001/062219

(56) Entgegenhaltungen:
- EP-A- 0 370 492
- EP-A- 0 873 746
- WO-A-00/33799
- WO-A-00/38639
- DE-A- 19 820 894
- DE-U- 29 908 464

## Beschreibung

Gegenstand der vorliegenden Erfindung ist ein Mittel zur Färbung von Fasern, insbesondere Keratinfasern (wie zum Beispiel menschlichen Haaren), das Indolderivate und Carbonylverbindungen enthält sowie ein Mehrkomponenten-Kit zur Färbung und späteren Entfärbung von Fasern, der sowohl Mittel zur Erzeugung einer Färbung auf der Faser als auch Mittel zur reduzierenden Entfernung der Färbung enthält.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen und ermöglichen eine Grauabdeckung von etwa 20 %.

Im allgemeinen waschen sich direktziehende und oxidative Tönungen nach einigen Haarwäschen heraus. Die Zeitdauer hängt unter anderem sehr stark von der Haarstruktur und der verwendeten Nuance ab. Oxidative Farben können teilweise mit der Zeit verblassen, verbleiben aber in der Regel bis zum nächsten Haarschnitt im Haar. Eine jederzeit mögliche Entfernung der Haarfärbung kann jedoch dann wünschenswert sein, wenn man eine besondere Farbe nur für einen bestimmten Zeitraum tragen will, oder eine Färbung dem Anwender nicht gefällt. Ebenso kann im Falle der Haarfärbung bei Erstverwendern die Möglichkeit einer schonenden und vollständigen Entfernung der Färbung die Angst vor einer zu drastischen Farbveränderung vermindern ("Färbung auf Probe").

Aus der EP-PS 0 847 749 ist die Verwendung einer Kombination von Diimino-isoindolinderivaten oder 3-Amino-isoindolonderivaten und Verbindungen mit primären oder sekundären Aminogruppen zur Färbung von Keratinfasern ohne Zusatz von Oxidationsmitteln bekannt. Ebenfalls ist es aus der DE-OS 43 35 623 bekannt, zur Färbung von Keratinfasern eine Kombination aus lndolinonderivaten und Verbindungen mit primären oder sekundären Aminogruppen, Heterozyklen oder aromatischen Hydroxyverbindungen einzusetzen. In der DE-OS 44 09 143 wird zudem die Verwendung von Isatinderivaten zur Färbung von Keratinfasern beschrieben. Aus der DE-OS 197 45 292 ist die Verwendung einer Kombination von Malonaldehydderivaten, wie zum Beispiel Malonaldehydbis-dialkylacetalen, und Aminen oder CH-aciden Verbindungen zur Färbung von Haaren ohne Zusatz von Oxidationsmitteln bekannt. Ebenfalls ist aus der DE-OS 197 17 280 die Verwendung einer Kombination von bestimmten heterozyklischen Aldehyden und Aminen oder CH-aciden Verbindungen zur Färbung von Haaren ohne Zusatz von Oxidationsmitteln bekannt.

Es besteht jedoch weiterhin ein großer Bedarf für Färbemittel, die unter milden Bedingungen sowohl intensive als auch schonende Färbungen mit einer breiten Nuancenpalette ermöglichen und -falls-gewünscht- zu einem beliebigen späteren Zeitpunkt wieder entfärbt werden können.

Aufgabe der vorliegenden Erfindung ist es daher, ein Färbesystem zur Verfügung zu stellen, das ohne Zusatz von Oxidationsmitteln (wie zum Beispiel Wasserstoffperoxid) zum einen eine schonende, intensive Färbung der Fasern im Gelb-, Braun-, Grün und Violettbereich mit guten Echtheitseigenschaften (Lichtechtheit, Waschechtheit, Reibechtheit) und zum anderen eine schonende und vollständige Entfernung dieser Fäbung zu jedem beliebigen Zeitpunkt ermöglicht.

Überraschenderweise wurde nunmehr gefunden, daß bei Verwendung eines ein Indolinderviat der Formel (I) oder ein 3H-lndoliumderivat der Formel (Ia) sowie eine Carbonylverbindung enthaltenden Färbemittels auf schonende Weise intensive Färbungen erzielt werden, welche zu einem beliebigen späteren Zeitpunkt wieder vollständig entfernt werden können.

Gegenstand der vorliegenden Erfindung ist daher ein Mittel zur Färbung von Fasern, wie zum Beispiel Wolle, Seide, Baumwolle oder Haaren und insbesondere keratinischen Fasern wie zum Beispiel menschlichen Haaren, welches durch Vermischen zweier Komponenten -falls erforderlich unter Zusatz eies Alkalisierungsmittels oder einer Säureerhalten wird, und dadurch gekennzeichnet ist, dass die eine Komponente (Komponente A2) mindestens eine Carbonylverbindung, insbesondere eine aromatische Aldehydverbindung, enthält und die andere Komponente (Komponente A1) mindestens ein Indolinderivat der Formel (I) oder ein 3H-Indolium-Derivat der Formel (Ia) enthält, 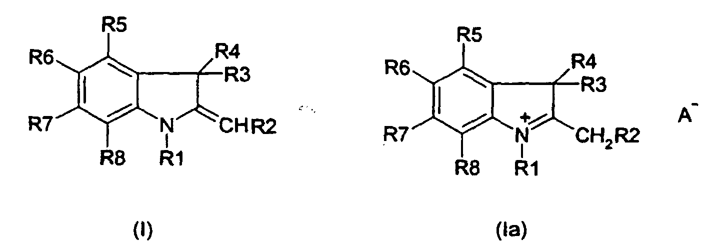 wobei in den Formeln (I) und (Ia) die Restgruppen R1 bis R8 und A die folgende Bedeutung haben:
**R1** ist gleich einer geradkettigen oder verzweigten C1- bis C8-Alkylgruppe, C1- bis C8-Monohydroxyalkylgruppe, C2- bis C8-Polyhydroxyalkylgruppe, C1- bis C8-Alkoxy-(C1- bis C8)alkylgruppe oder Thio-(C1- bis C8)-alkylgruppe, einer -(CH₂)ₘ-X-(CH₂)ₙ-Y-(CH₂)ₚ-R^{a}-Gruppe, einer -(CH₂)ₙ-X-R^{a}-Gruppe, einer -(CH₂)ₘ-Y-(CH₂)ₙ-X-(CH₂)ₚ-R^{a}-Gruppe, einer -(CH₂)ₘ-CO-(CH₂)ₚ-X-R^{a}-Gruppe, einer -(CH₂)ₚ-R^{a} -Gruppe, einer -(CH₂)ₘ-X-(CH₂)ₚ-CO-Y-R^{a}-Gruppe, oder gleich 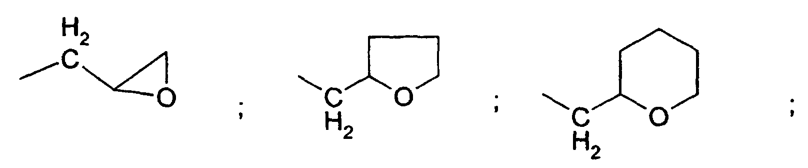 wobei X und Y unabhängig voneinander gleich einem Sauerstoffatom, einem Schwefelatom oder einer NR^{b}-Gruppe sind, R^{a} und R^{b} unabhängig voneinander gleich einem Wasserstoffatom, einem gegebenenfalls substituierten aromatischen Carbozyklus oder Heterocyclus oder einer geradkettigen oder verzweigten C1- bis C8-Alkylgruppe sind,
m und n unabhängig voneinander gleich einer ganzen Zahl von 1 bis 6 sind und p gleich einer ganzen Zahl von 0 bis 6 ist;
**R2** ist gleich einem Wasserstoffatom oder einer geradkettigen C1- bis C6-Alkylgruppe;
**R3** und **R4** sind unabhängig voneinander gleich einer geradkettigen oder verzweigten C1- bis C4-Alkylgruppe (insbesondere einer Methylgruppe), einer -(CH₂)ₙ-R^{c}, -(CH₂)ₘ-CHR^{c}-X-(CH₂)ₙ-R^{c}-Gruppe, einer -(CH₂)ₙ-CO-R^{c}-Gruppe, einer -(CH₂)ₙ-CO-XR^{c}-Gruppe, einer -(CH₂)ₙ-CN -Gruppe, einer -(CH₂)ₙ-CH=C(CH₃)₂ -Gruppe, einer -(CH₂)ₘ-X-CHR^{c}-(CH₂)ₙ-R^{c} -Gruppe oder einer -(CH₂)ₙCH=CH -Gruppe ist, wobei X gleich einem Sauerstoffatom, einem Schwefelatom oder einer NR^{b}-Gruppe ist, m und n unabhängig voneinander gleich 1 bis 6 sind, und R^{c} für ein Wasserstoffatom, einen gegebenenfalls substituierten aromatischen Carbozyklus oder Heterocyclus oder eine geradkettige oder verzweigte C1- bis C6-Alkylgruppe steht; mit der Maßgabe, dass die Reste R3 und R4 auch gemeinsam, verbunden über eine (CH₂)ₙ-Gruppe (mit n=1-3), mit dem 3H-Kohlenstoff eine Spiroverbindung bilden können;
**R5, R6, R7** und **R8** sind unabhängig voneinander gleich einem Wasserstoffatom, einer geradkettigen oder verzweigten C1- bis C4-Alkylgruppe oder C1- bis C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethylgruppe, einer -CHO-Gruppe, einer -COR^{d}-Gruppe, einer -COOH-Gruppe, einer -CO₂R^{d}-Gruppe, einer -OCOR^{d}-Gruppe, einer -OCH₂ArylGruppe, einer -SO₂NH₂-Gruppe, einer -NH₂-Gruppe, einer -NH₃⁺ -Gruppe, einer -NHR^{d}-Gruppe, einer -NH₂R^{d+} -Gruppe, einer -N(R^{d})₂-Gruppe, einer -N(R^{d})₃⁺-Gruppe, einer -NHCOR^{d} -Gruppe, einer -NHCOOR^{d} -Gruppe, einer -CH₂NH₂ -Gruppe, einer -CH₂NHR^{d} -Gruppe, einer -CH₂N(R^{d})₂-Gruppe, einer -CO₂CF₃ -Gruppe, einer -PO(OR^{d})₂ -Gruppe, einer -SO₂CHF₂-Gruppe, einer -SO₂CF₃-Gruppe, einer -SO₂R^{d}- Gruppe oder einer -SR^{d} -Gruppe, wobei R^{d} gleich einem Wasserstoffatom, einem gegebenenfalls substituierten aromatischen Carbozyklus oder Heterocyclus oder einer C1- bis C6-Alkylgruppe ist, unter der Bedingung, dass (i) mindestens einer der Reste **R5** bis **R8** von Wasserstoff verschieden ist, und (ii) das Indolinderivat der Formel (I) nicht gleich 5-Chloro-2-methylen-1,3,3-trimethylindolin ist, wenn die Carbonylverbindung gleich 3,4-Dihydroxybenzaldehyd oder 4-Hydroxybenzaldehyd ist;
**A**^{**-**} ist gleich einem Anion einer organischen oder anorganischen Säure.

Vorzugsweise ist **A**^{**-**} gleich Chlorid, Bromid, Jodid, Hydrogensulfat, Sulfat, Toluolsulfonat, Benzolsuifonat, Monomethylsulfat, Hexafluorphosphat, Hexafluorantimonat, Tetrafluorborat, Tetraphenylborat, Formiat, Acetat oder Propionat, wobei das Chloridion, dasTetrafluoroboration, das Acetation und das Hydrogensulfation besonders bevorzugt sind.

Unter den Verbindungen der Formeln (I) und (la) sind die folgenden Verbindungen bevorzugt:
1,3,3,4-Tetramethyl-2-methylen-indolin sowie dessen Salze,
1,3,3,5-Tetramethyl-2-methylen-indolin sowie dessen Salze,
1,3,3,6-Tetramethyl-2-methylen-indolin sowie dessen Salze,
1,3,3,7-Tetramethyl-2-methylen-indolin sowie dessen Salze,
1,3,3,6,7-Pentamethyl-2-methylen-indolin sowie dessen Salze,
1,3,3,5,7-Pentamethyl-2-methylen-indolin sowie dessen Salze,
1,3,3,4,7-Pentamethyl-2-methylen-indolin sowie dessen Salze,
5-Fiuoro-1,3,3-trimethyl-2-methylen-indolin sowie dessen Salze,
5-Isopropyl-1,3,3-trimethyl-2-methylen-indolin sowie dessen Salze,
5-Hydroxy-1,3,3-trimethyl-2-methylen-indolin sowie dessen Salze,
5-Methoxy-1,3,3-trimethyl-2-methylen-indolin sowie dessen Salze,
5-Nitro-1,3,3-trimethyl-2-methylen-indolin sowie dessen Salze,
5-Amino-1,3,3-trimethyl-2-methylen-indolin sowie dessen Salze,
5-N-acetylamino-1,3,3-trimethyl-2-methylen-indolin sowie dessen Salze,
6-Hydroxy-1,3,3-trimethyl-2-methylen-indolin sowie dessen Salze,
6-Methoxy-1,3,3-trimethyl-2-methylen-indolin sowie dessen Salze
5-Methoxy-6-nitro-1,3,3-trirnethyl-2-methylen-indolin sowie dessen Salze,
5-Methoxy-6-amino-1,3,3-trimethyl-2-methylen-indolin sowie dessen Salze,
5-Methoxy-6-N-acetylamino-1,3,3-trimethyl-2-methylen-indolin sowie dessen Salze,
5,6-Dihydroxy-1,3,3-trimethyl-2-methylen-indolin sowie dessen Salze,
5,6-Dimethoxy-1,3,3-trimethyl-2-methylen-indolin sowie dessen Salze,
5,6-Methylendioxy-1,3,3-trimethyl-2-methylen-indolin sowie dessen Salze,
4,5-Dihydroxy-1,3,3-trimethyl-2-methylen-indolin sowie dessen Salze,
5,7-Dihydroxy-1,3,3-trimethyl-2-methylen-indolin sowie dessen Salze,
5-Amino-6-methoxy-1,3,3-trimethyl-2-methylen-indolin sowie dessen Salze,
5-Amino-7-hydroxy-1,3,3-trimethyl-2-methylen-indolin sowie dessen Salze,
5-Hydroxy-7-amino-1,3,3-trimethyl-2-methylen-indolin sowie dessen Salze,
5-Hydroxy-7-N-acetylamino-1,3,3-trimethyl-2-methylen-indolin sowie dessen Salze,
1-Methyl-3-spirocyclohexyl-5-hydroxy-2-methylen-indolin sowie dessen Salze, 1-Methyl-3-spirocyclohexyl-5-methoxy-2-methylen-indolin sowie dessen Salze; wobei das 1,2,3,3,5-Pentamethyl-3H-indolium-jodid,
1,2,3,3,7-Pentamethyl-3H-indolium-tetrafluoroborat,
1,2,3,3,6,7-Hexamethyl-3H-indolium-tetrafluoroborat,
1,2,3,3,5,7-Hexamethyl-3H-indolium-tetrafluoroborat,
1,2,3,3,4,7-Hexamethyl-3H-indolium-tetrafluoroborat,
5-Fluoro-1,2,3,3-tetramethyl-3H-indolium-jodid,
5-lsopropyl-1,2,3,3-tetramethyl-3H-indolium-jodid,
5-Nitro-1,3,3-trimethyl-2-methylen-indolin,
und insbesondere das 5-Methoxy-1,2,3,3-tetramethyl-3H-indolium-jodid,
5-Methoxy-6-nitro-1,2,3,3-tetramethyl-3H-indolium-chiorid,
5-Hydroxy-1,2,3,3-tetramethyl-3H-indolium-jodid und
5-N-acetylamino-1,2,3,3-tetramethyl-3H-indolium-acetat besonders bevorzugt sind.

Die in dem erfindungsgemässen Mittel verwendeten Verbindungen der Formeln (I) und (Ia) sind aus der Literatur bekannt oder durch literaturbekannte Standardsyntheseverfahren, wie sie beispielsweise in der Dissertation von Andreas Leiminer, Universität Regensburg (1995); der DE-OS 1 949 716 oder der US-PS 3 865 837 beschrieben werden, herstellbar. Über literaturbekannte elektrophile Substitutionsreaktionen am aromatischen Kern können zusätzlich weitere neue Substituenten, wie zum Beispiel eine Nitrofunktion, eingeführt werden. In diesem Zusammenhang sei insbesondere auf das von D.J. Gale, J.F.K. Wilshire in J. Soc. Dyers Colour ; 1974, Seiten 97-100 beschriebene Syntheseverfahren hingewiesen. Durch anschliessende Behandlung mittels Reduktions- oder Oxidationsmitteln beziehungsweise mit Hilfe von geeigneten Schutzgruppenadditions- oder-eliminationsreaktionen können die eingeführten Substituenten umfunktionalisiert werden, wodurch weitere Verbindungen der allgemeinen Formeln (I) und (Ia) erhältlich sind.

Als geeignete Carbonylverbindungen sind insbesondere die folgenden Aldehyde zu nennen: Vanillin (4-Hydroxy-3-methoxybenzaldehyd), Isovanillin (3-Hydroxy-4-methoxy-benzaldehyd), 3,4-Dihydroxybenzaldehyd, 4-Hydroxybenzaldehyd, 3,5-Dimethoxy-4-hydroxybenzaldehyd, 4-Dimethylaminobenzaldehyd, 4-Methyl-5-imidazolcarboxaldehyd, 4- Dimethylamino-zimtaldehyd, 4-Hydroxy-2-methoxybenzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-rnethoxybenzaldehyd, 2,4-Dihydroxybenzaldehyd, 3,4-Dihydroxy-benzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxybenzaldehyd, 2,5-Dimethoxy-benzaldehyd, 3,5-Dimethoxybenzaldehyd, 3,4-Dimethoxybenzaldehyd, lndol-3-carbaldehyd, Benzol-1,4-dicarbaldehyd, 4-Ethoxybenzaldehyd, 2-Methyl-1,4-naphthochinon, 4-Carboxy-benzaldehyd, 4-Hydroxy-3-methoxyzimtaldehyd, 3,5-Dimethoxy-4-hydroxy-zimtaldehyd, 3-Methoxy-4-(1-pyrrolidinyl)-benzaldehyd, 4-Diethylamino-3-methoxybenzaidehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1(4H)-bebzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylaminobenzaldehyd, N-Ethylcarbazol-3-aldehyd, 4-Diethylamino-2-hydroxybenzaldehyd, 3,4-Dimethoxy-5-hydroxybenzaldehyd, 5-(4-(Diethylamino)phenyl)-2,4-pentadienal, 2,3-Thiophendicarboxaldehyd, 2,5-Thiophendicarboxaldehyd, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

Die Verbindungen der Formeln (I) und (Ia) werden bis kurz vor der Anwendung von den Carbonylverbindungen getrennt aufbewahrt. Das erfindungsgemäße Färbemittel besteht in der Regel aus einer Mischung der beiden Komponenten A1 und A2, nämlich einer Farbträgermasse (A1), welche die Verbindungen der Formel (I) und/oder (Ia) und gegebenenfalls direktziehende Farbstoffe enthält, und einer weiteren Farbträgermasse (A2), welche die Carbonylverbindung und gegebenenfalls direktziehende Farbstoffe enthält. Diese beiden Komponenten werden unmittelbar vor der Anwendung zu einem gebrauchsfertigen Färbemittel vermischt und sodann auf die zu färbende Faser aufgetragen. Selbstverständlich ist es auch möglich, dass eine oder beide Komponenten aus mehreren Einzelkomponenten bestehen, welche vor der Anwendung miteinander vermischt werden.

Die Verbindungen der Formeln (I) und (Ia) und die Carbonylverbindungen sind in der jeweiligen Farbträgermasse (Komponente A1 beziehungsweise Komponente A2) jeweils in einer Gesamtmenge von etwa 0,02 bis 20 Gewichtsprozent, vorzugsweise 0,2 bis 10 Gewichtsprozent, enthalten, wobei in dem durch Vermischen der Komponenten A1 und A2 erhaltenen gebrauchsfertigen Färbemittel die Verbindungen der Formeln (I) und (Ia) und die Carbonylverbindung jeweils in einer Gesamtmenge von etwa 0,01 bis 10 Gewichtsprozent, vorzugsweise 0,1 bis 5 Gewichtsprozent, enthalten sind.

Weiterhin kann das erfindungsgemäße Färbemittel gegebenenfalls zusätzlich übliche, physiologisch unbedenkliche, direktziehende Farbstoffe aus der Gruppe der Nitrofarbstoffe, Azofarbstoffe, Chinonfarbstoffe und Triphenylmethanfarbstoffe enthalten.

Die direktziehenden Farbstoffe können in der Komponente A1 und der Komponente A2 jeweils in einer Gesamtenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten A1 und A2 erhaltenen gebrauchsfertigen Färbemittel etwa 0,01 bis 10 Gewichtsprozent, vorzugsweise 0,1 bis 5 Gewichtsprozent, beträgt.

Die Zubereitungsform für das gebrauchsfertige Färbemittel sowie die Komponenten A1 und A2 kann beispielsweise eine Lösung, insbesondere eine wäßrige oder wäßrig-alkoholische Lösung sein. Weitere geeignete Zubereitungsformen sind eine Creme, ein Gel, ein Aerosolschaum oder eine Emulsion. Ihre Zusammensetzung stellt eine Mischung der Verbindungen der Formeln (I) und (Ia) und/oder der Carbonylverbindungen mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Der pH-Wert des gebrauchsfertigen Färbemittels beträgt in der Regel etwa 3 bis 11, vorzugsweise etwa 6 bis 11, wobei ein pH-Wert von 6,5 bis 8,5 besonders bevorzugt ist. Der pH-Wert des gebrauchsfertigen Färbemittels ergibt sich bei der Mischung der enaminhaltigen Komponente A1 mit der carbonylhaltigen Komponente A2 in Abhängigkeit vom pH-Wert der Komponenten A1 und A2 sowie dem Mischungsverhältnis dieser beiden Komponenten. Falls erforderlich kann nach dem Vermischen der Komponenten A1 und A2 der pH-Wert des gebrauchsfertigen Färbemittels durch den Zusatz eines alkalisierenden Mittels oder einer Säure auf den gewünschten Wert eingestellt werden.

Zur Einstellung des pH-Wertes des gebrauchsfertigen Mittels sowie der Komponente A1 oder A2 können alkalisierende Mittel wie zum Beispiel Alkanolamine, Alkylamine, Alkalihydroxide oder Ammoniumhydroxid und Alkalicarbonate oder Ammoniumcarbonate oder Säuren wie zum Beispiel Milchsäure, Essigsäure, Weinsäure, Phosphorsäure, Salzsäure, Zitronensäure, Ascorbinsäure und Borsäure, verwendet werden.

Das gebrauchsfertige Färbemittel wird unmittelbar vor der Anwendung durch Vermischen der die Verbindungen der Formeln (I) und (Ia) enthaltenden Komponente A1 mit der die Carbonylverbindung enthaltenden Komponente A2 (gegebenenfalls unter Zusatz eines alkalisierenden Mittels oder einer Säure) hergestellt und sodann auf die Faser aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschliessend wird die Faser mit Wasser gespült und gegebenenfalls mit einem Shampoo gewaschen.

Das erfindungsgemässe Färbemittel ermöglicht eine schonende, gleichmässige und dauerhafte Färbung der Fasern, insbesondere von Keratinfasem, wie zum Beispiel Haaren. Überraschenderweise können diese Färbungen zu einem beliebigen Zeitpunkt schnell und schonend durch Reduktionsmittel wieder vollständig entfärbt werden.

Ein weiter Gegenstand der vorliegenden Erfindung ist daher ein Mehrkomponenten-Kit zur Färbung und späteren Entfärbung von Fasern, wie zum Beispiel Wolle, Seide, Baumwolle oder Haaren und insbesondere menschlichen Haaren, welcher dadurch gekennzeichnet ist, dass er das erfindungsgemässe Färbemittel (A) und eine entfärbende Komponente (B) enthält, wobei die Komponete B als entfärbendes Agenz mindestens ein Sulfit, beispelsweise ein Ammoniumsulfit, Alkalisulfit oder Erdalkalisulfit, insbesondere Natriumsulfit oder Ammoniumsulfit, enthält.

Die Gesamtmenge an Sulfiten in der Komponente B beträgt etwa 0,1 bis 10 Gewichtsprozent, vorzugsweise 2 bis 5 Gewichtsprozent.

Das Mittel zur Entfärbung der mit dem Färbemittel A gefärbten Fasern (im folgenden "Entfärbemittel" genannt) kann als wässrige oder wässrigalkoholische Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann neben der Pulverform zum Schutz vor Staubbildung auch als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranutat beziehungsweise der Brausetablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 3 bis 8, insbesondere 4 bis 7. Erforderlichenfalls kann der gewünschte pH-Wert durch Zugabe von geeigneten Säuren, beispielsweise α-Hydroxycarbonsäuren wie Milchsäure, Weinsäure, Zitronensäure oder Äpfelsäure, Phosphorsäure, Essigsäure, Glycolsäure Salicylsäure. Glutathion oder Gluconsäurelacton, oder aber alkalisierenden Mitteln wie Alkanolaminen, Alkylaminen, Alkalihydroxiden, Ammoniumhydroxid, Alkalicarbonaten, Ammoniumcarbonaten oder Alkaliphosphaten, eingestellt werden.

Die Einwirkungszeit des Entfärbemittels beträgt je nach zu entfärbender Färbung und Temperatur (etwa 20 bis 50 Grad Celsius) 5 bis 60 Minuten, insbesondere 15 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen.

Obwohl die Komponente B zur Entfärbung von mit dem Färbemittel A gefärbten Haaren, insbesondere menschlichen Haaren, besonders gut geeignet ist, kann die Komponente B prinzipiell auch zur Entfärbung von anderen mit dem Färbemittel A gefärbten natürlichen oder synthetischen Fasern, wie zum Beispiel Baumwolle, Wolle, Seide, Viskose, Nylon, Celluloseacetat, verwendet werden.

Die nachfolgenden Beispiele sollen den Gegenstand näher erläutern, ohne ihn auf diese Beispiele zu beschränken.

### Beispiele

### Beispiele 1.1 bis 1.12: Synthese der Indolderivate der Formel (I)/(Ia)

### Allgemeine Synthesevorschriften

### 1a) Allgemeine Vorschrift zur Herstellung der 3H-Indolderivate der Formel (I)/(Ia)

Das substituierte Arylhydrazin (1 Äquivalent) wird in Ethanol bei 25 °C gelöst. Dazu werden 1,2 Äquivalente des Alkylketons gegeben und anschliessend wird 3 Stunden lang unter Rückfluss erhitzt. Nach dem Abkühlen auf etwa 60 °C wird zur gebildeten Arylhydrazon-Lösung konzentrierte Schwefelsäure zugetropft und weitere 5 Stunden unter Rückfluss erhitzt. Die Reaktionslösung wird auf 25 °C abgekühlt und unter vermindertem Druck auf das halbe Ursprungsvolumen eingeengt. Sodann gibt man destilliertes Wasser zu dieser Reaktionslösung und stellt die Lösung mit einer viermolaren Natronlauge basisch ein. Die entstandene Emulsion wird mit Diethylether extrahiert. Die vereinigten organischen Phasen werden über Na₂SO₄ getrocknet, filtriert und eingeengt. Das resultierende Oel wird mittels Kugelrohrdestillation gereinigt.

### 1b) Allgemeine Vorschrift zur N-Alkylierung der 3H-Indolderivate aus Stufe 1a

### Variante 1:

Das in 1a) erhaltene Zwischenprodukt (1 Äquivalent) wird in Chloroform gelöst und mit Methyljodid (1,1 bis 2,1 Äquivalente) versetzt. Die erhaltene Lösung wird sodann unter Lichtausschluss und unter Argonatmosphere 24 Stunden lang bei 25 °C gerührt. Anschliessend wird tert-Butylmethylether zu dieser Lösung hinzugegeben, die gelbe Fällung abgesaugt und aus Ethanol umkristallisiert.

### Variante 2:

Eine Suspension des in 1a) erhaltenen Zwischenproduktes (1 Äquivalent) in Methyljodid (5 Äquivalente) wird 4 Stunden lang mit Ultraschall (120 W) gerührt. Der gebildete Feststoff wird abfiltriert, mit tert-Butylmethylether gewaschen und aus Ethanol umkristallisiert.

### Variante 3:

Das in 1a) erhaltene Zwischenprodukte (1 Äquivalent) wird in Methanol gelöst, mit Methyljodid (2 Äquivalente) versetzt und 12 Stunden lang unter Rückfluss erwärmt, wobei die Reaktionslösung unter Lichtausschluss und Argonatmosphere gehalten wird. Die Reaktionslösung wird anschliessend unter vermindertem Druck vollständig eingeengt, der Rückstand wird mit Essigsäureethylester gewaschen und aus Methanol umkristallisiert.

### Variante 4:

Das in 1a) erhaltene Zwischenprodukt (1 Äquivalent) wird in 1,2-Dichlorethan gelöst, mit Trimethyloxonium-tetrafluoroborat (1,2 Äquivalente) versetzt und 6 Stunden unter Rückfluss erwärmt. Das Produkt fällt aus, wenn man die Reaktionsmischung auf 4 °C kühlt oder Essigsäureethylester zugibt. Der erhaltene Feststoff wird abfiltriert, mit wenig Essigsäureethylester gewaschen und aus Methanol umkristallisiert.

### 1c) Allgemeine Vorschrift zur Demethylierung des Arylmethylethers

Der Arylmethylether wird in Essigsäure gelöst und mit 48%iger Bromwasserstofflösung versetzt. Die Reaktionsmischung wird 8 Stunden lang unter Rückfluss erhitzt und anschliessend über Nacht bei Raumtemperatur gerührt. Die Reaktionslösung wird mit 20%iger Natronlauge neutralisiert, mit gesättigter NatriumhydrogencarbonatLösung basisch gestellt (pH = 8,0) und mit Essigsäureethylester extrahiert. Die vereinigten organischen Phasen werden mit Wasser und gesättigter Natriumchtorid-Lösung gewaschen, über MgSO₄ getrocknet, filtriert und vollständig eingeengt. Der erhaltene Feststoff wird aus Essigsäureethylester umkristallisiert.

### 1d) Allgemeine Vorschrift zur Nitrierung der Verbindungen der Formel (I) oder (Ia)

Zu eisgekühlter konzentrierter Schwefelsäure gibt man portionsweise die Verbindung der Formel (I) oder (Ia), wobei eine Reaktionstemperatur von 10 °C nicht überschritten werden darf. Zur entstandenen Lösung tropft man Nitriersäure (100%ige HNO₃ gelöst in 95%iger H₂SO₄), wobei die Reaktionstemperatur ebenfalls unter 10 °C gehalten werden muss. Man rührt anschliessend 3 Stunden lang bei 5 °C und giesst sodann die Reaktionslösung auf Eis und stellt die wässrige Phase mit Natronlauge basisch ein. Der resultierende Feststoff wird mit Wasser gewaschen und anschliessend in tert-Butylmethylether aufgenommen. Die organische Phase wird mit Wasser gewaschen, über Na₂SO₄ getrocknet, filtriert und vollständig eingeengt. Das Rohprodukt wird aus Hexan/Methylenchlorid umkristallisiert oder in heissem Acetonitril und wenig 3molarer Salzsäure (in Ethanol) gelöst und mit Wasser ausgefällt.

### 1e) Allgemeine Vorschrift zur Reduktion der Nitroverbindungen zu den entsprechenden Arylaminen mit anschliessender N-Acetylierungsreaktion

### Reduktion der arylischen Nitrofunktion:

Die nach 1d) hergestellte Nitroverbindung wird in 32%iger wässriger Salzsäure gelöst und mit SnCl₂ x 2 H₂O versetzt. Die Reaktionsmischung wird 1 bis 3 Stunden lang unter Rückfluss erhitzt und anschliessend auf 25 °C abgekühlt. Nachdem man die Reaktionslösung auf Eis gegossen hat, wird die Reaktionslösung mit einer 4 molaren Natronlauge basisch gestellt und anschliessend wird mit tert-Butylmethylether extrahiert. Die vereinigten organischen Phasen werden über Na₂SO₄ getrocknet, filtriert, mit einer 3molaren Salzsäure-Lösung (in Ethanol) sauer gestellt und vollständig unter vermindertem Druck eingeengt. Der Rückstand wird in wenig 3molarer Salzsäure-Lösung (in Ethanol) gelöst und zu kaltem tert-Butylmethylether gegeben, wobei das Produkt ausfällt und durch Filtration unter Stickstoff isoliert wird.

### N-Acetylierung:

Das vorgenannte Produkt wird in Essigsäure gelöst und mit Essigsäureanhydrid versetzt. Die Reaktionsmischung wird sodann bei 25 °C 1 bis 3 Stunden lang kräftig gerührt und anschliessend zu tert-Butylmethylether gegeben. Die entstandene Fällung wird abgesaugt, mit Ether gewaschen und im Vakuum getrocknet.

### Beispiel 1.1: Synthese von 1,2,3,3,5-Pentamethyl-3H-indolium-jodid

Die Herstellung erfolgt gemäss der allgemeinen Synthesevorschrift 1a), wobei 3,27 g p-Tolylhydrazin und 2,87 g lsopropylmethylketon in 110 ml Ethanol und 3,0 ml 97%iger Schwefelsäure eingesetzt werden.
Das resultierende Oel wird mittels Kugelrohrdestillation im Vakuum (0,02-0,07 mbar; 100-105 °C) gereinigt.
Ausbeute (Zwischenprodukt): 3,18 g (68 % der Theorie) 2,3,3,5-Tetramethyl-3H-indol
1,0 g des so erhaltenen Zwischenproduktes wird gemäss Variante 2 in 1b) mit 4,18 g Methyljodid alkyliert.
Ausbeute: 1,11 g (61% der Theorie) 1,2,3.3,5-pentamethyl-3H-indolium-jodid
Schmelzpunkt: 224-227°C
¹H-NMR (CD₃OD): δ = 1,59 ppm (s, 6H); 2,50 ppm (s, 3H); 4,03 ppm (s, 3H); 4,83 ppm (s, 3H); 7,45 ppm (dd, ³J = 8 Hz, ⁴J = nicht aufgelöst, 1H); 7,59 ppm (d, ⁴J = nicht aufgelöst, 1H), 7,70 ppm (d, ³J = 8Hz, 1H). FAB-Massenspektrum: M⁺ = 188,30 (100% rel. Intensität)

| Elementaranalyse: C₁₃H₁₈NJ (315,19) | | | |
|---|---|---|---|
| | %C | %H | %N |
| ber. | 49,54 | 5,76 | 4,74 |
| gef. | 49,80 | 5,77 | 4,30 |

### Beispiel 1.2: Synthese von 1,2,3,3,7-Pentamethyl-3H-indoliumtetrafluoroborat

Die Herstellung erfolgt gemäss der allgemeinen Synthesevorschrift 1a), wobei 3,39 g o-Tolylhydrazin und 3,0 g lsopropylmethylketon in 120 ml Ethanol und 3,1 ml 97%iger Schwefelsäure eingesetzt werden.
Das resultierende Oel wird mittels Kugelrohrdestillation im Vakuum (0,02-0,07 mbar; 100-105 °C) gereinigt.
Ausbeute (Zwischenprodukt): 3,0 g (62 % der Theorie) 2,3,3,7-Tetramethyl-3H-indol
0,50 g des so erhaltenen Zwischenproduktes wird gemäss Variante 4 in 1b) mit 0,51 g Trimethyloxonium-tetrafluoroborat und 1 ml 1,2-Dichlorethan alkyliert.
Ausbeute: 0,43 g (54 % der Theorie) 1,2,3,3,7-Pentamethyl-3H-indoliumtetrafluoroborat
¹H-NMR(D₆-DMSO): δ =1,50 ppm (s, 6H); 2,74 ppm (s, 3H); 2,75 ppm (s, 3H); 4,11 ppm (s, 3H); 7,35 ppm (dd, ³J = 7,5 Hz , ⁴J = nicht aufgelöst, 1H), 7,46 ppm (dd, ³J = 7,5 Hz , ³J = 7,5 Hz, 1H); 7,60 ppm (dd, ³J = 7,5 Hz, ⁴J = nicht aufgelöst, 1H);
FAB-Massenspektrum: M⁺ = 188,3 (100% rel. Intensität)

| Elementaranalyse: C₁₃H₁₈NBF₄ (275,10) | | | | |
|---|---|---|---|---|
| | %C | %H | %N | %F |
| ber. | 56,76 | 6,60 | 5,09 | 27,62 |
| gef. | 56,52 | 6,48 | 5,21 | 27,57 |

### Beispiel 1.3: Synthese von 1,2,3,3,6,7-Hexamethyl-3H-indoliumtetrafluoroborat

Die Herstellung erfolgt gemäss der allgemeinen Synthesevorschrift 1a), wobei 3,76 g 2,3-Dimethylphenylhydrazin und 2,97 g Isopropylmethylketon in 120 ml Ethanol und 3,1 ml 97%iger Schwefelsäure eingesetzt wird. Das resultierende Oel wird mittels Kugelrohrdestillation im Vakuum (0,02-0,07 mbar; 100-105 °C) gereinigt.
Ausbeute (Zwischenprodukt): 1,90 g (37 % der Theorie) 2,3,3,6,7-Pentamethyl-3H-indol
1,0 g des so erhaltenen Zwischenproduktes wird gemäss Variante 4 in 1b) mit 0,95 g Trimethyloxonium-tetrafluoroborat und 4 ml 1,2-Dichlorethan alkyliert.
Ausbeute: 0,49 g (32% der Theorie) 1,2,3,3,6,7-Hexamethyl-3H-indoliumtetrafluoroborat
¹H-NMR (D₆-DMSO): δ = 1,45 ppm (s, 6H); 2,37 ppm (s, 3H); 2,61 ppm (s, 3H); 2,72 ppm (s, 3H); 4,11 ppm (s, 3H); 7,40 ppm (d, ³J = 8 Hz, 1H); 7,49 ppm (d, ³J = 8 Hz, 1H)
FAB-Massenspektrum: M⁺ = 202,3 (100% rel. Intensität)

| Elementaranalyse: C₁₄H₂₀NBF₄ (289,12) | | | | |
|---|---|---|---|---|
| | %C | %H | %N | %F |
| ber. | 58,16 | 6,97 | 4,84 | 26,28 |
| gef. | 57,86 | 6,88 | 4,78 | 26,15 |

### Beispiel 1.4: Synthese von 1,2,3,3,5,7-Hexamethyl-3H-indoliumtetrafluoroborat

Die Herstellung erfolgt gemäss der allgemeinen Synthesevorschrift 1a), wobei 4,57 g 2,4-Dimethylphenylhydrazin und 3,08 g lsopropylmethylketon in 80 ml Ethanol und 3,2 ml 97%iger Schwefelsäure eingesetzt wird. Das resultierende Oel wird mittels Kugelrohrdestillation im Vakuum (0,02-0,07 mbar; 100-105 °C) gereinigt.
Ausbeute (Zwischenprodukt): 2,67 g (50 % der Theorie) 2,3,3,5,7-Pentamethyl-3H-indol
1,0 g des so erhaltenen Zwischenproduktes wird gemäss Variante 4 in 1b) mit 0,95 g Trimethyloxonium-tetrafluoroborat und 4 ml 1,2-Dichlorethan alkyliert.
Ausbeute: 0,79 g (51 % der Theorie) 1,2,3,3,5,7-Hexamethyl-3H-indoliumtetrafluoroborat
¹H-NMR (D₆-DMSO): δ = 1,45 ppm (s, 6H); 2,36 ppm (s, 3H); 2,69 ppm (s, 3H); 2,70 ppm (s, 3H); 4,07 ppm (s, 3H); 7,18 ppm (s, 1H); 7,44 ppm (s, 1H)
FAB-Massenspektrum: M⁺ = 202,3 (100% rel. Intensität)

| Elementaranalyse: C₁₄H₂₀NBF₄ (289,12) | | | | |
|---|---|---|---|---|
| | %C | %H | %N | %F |
| ber. | 58,16 | 6,97 | 4,84 | 26,28 |
| gef. | 57,94 | 6,86 | 4,81 | 26,32 |

### Beispiel 1.5: Synthese von 1,2,3,3,4,7-Hexamethyl-3H-indoliumtetrafluoroborat

Die Herstellung erfolgt gemäss der allgemeinen Synthesevorschrift 1a), wobei 3,70 g 2,5-Dimethylphenylhydrazin und 2,93 g Isopropylmethylketon in 80 ml Ethanol und 3,1 ml 97%iger Schwefelsäure eingesetzt wird. Das resultierende Oel wird mittels Kugelrohrdestillation im Vakuum (0,02-0,07 mbar; 100-105 °C) gereinigt.
Ausbeute (Zwischenprodukt): 3,58 g (70 % der Theorie) 2,3,3,4,7-Pentamethyl-3H-indol
1,0 g des so erhaltenen Zwischenproduktes wird gemäss Variante 4 in 1b) mit 0,95 g Trimethyloxonium-tetrafluoroborat und 4 ml 1,2-Dichlorethan alkyliert.
Ausbeute: 0,83 g (53 % der Theorie) 1,2,3,3,4,7-Hexamethyl-3H-indoliumtetrafluoroborat
¹H-NMR (D₆-DMSO): δ = 1,53 ppm (s, 6H); 2,47 ppm (s, 3H); 2,70 ppm (s, 3H); 2,72 ppm (s, 3H); 4,07 ppm (s, 3H); 7,23 ppm (d, ³J = 8 Hz, 1H); 7,27 ppm (d, ³J = 8 Hz, 1H)
FAB-Massenspektrum: M⁺ = 202,3 (100% rel. Intensität)

| Elementaranalyse: C₁₄H₂₀NBF₄ (289,12) | | | | |
|---|---|---|---|---|
| | %C | %H | %N | %F |
| ber. | 58,16 | 6,97 | 4,84 | 26,28 |
| gef. | 58,10 | 6,86 | 4,80 | 26,11 |

### Beispiel 1.6: Synthese von 5-Methoxy-1,2,3,3-tetramethyl-3H-indolium-jodid

Die Herstellung erfolgt gemäss der allgemeinen Synthesevorschrift 1a), wobei 8,0 g 4-Methoxyphenylhydrazin und 6,23 g Isopropylmethylketon in 120 ml Ethanol und 11,3 g 97% iger Schwefelsäure eingesetzt wird.
Das resultierende Oel wird mittels Kugelrohrdestillation im Vakuum (0,02-0,07 mbar; 105-110 °C) gereinigt.
Ausbeute (Zwischenprodukt): 6,34 g (58 % der Theorie) 5-Methoxy-2,3,3-trimethyl-3H-indol
1,0 g des so erhaltenen Zwischenproduktes wurde gemäss Variante 1 in 1b) mit 1,59 g Methyljodid und 7,2 ml Chloroform alkyliert.
Ausbeute: 1,11 g (58 % der Theorie) 1,2,3,3,5-Pentamethyl-3H-indolium-jodid
¹H-NMR (CD₃OD): δ =1,59 ppm (s, 6H); 3,91 ppm (s, 3H); 4,02 ppm (s, 3H); 4,85 ppm (s, 3H); 7,15 ppm (dd, ³J = 9Hz, ⁴J=2Hz, 1H); 7,35 ppm (d, ⁴J = 2Hz, 1H) 7,73 ppm (d, ³J = 9 Hz, 1H).
FAB-Massenspektrum: M⁺= 204,0 (100% rel. lnt.), 188 (44% rel. lnt.)

| Elementaranalyse: C₁₃H₁₈NOJ (331,20) | | | | |
|---|---|---|---|---|
| | %C | %H | %N | %O |
| ber. | 47,15 | 5,48 | 4,23 | 4,83 |
| gef. | 47,30 | 6,10 | 4,30 | 4,90 |

### Beispiel 1.7: Synthese von 5-Fluoro-1,2,3,3-tetramethyl-3H-indolium-jodid

Die Herstellung erfolgt gemäss der allgemeinen Synthesevorschrift 1a), wobei 3,0 g 4-Fluorophenylhydrazin und 2,48 g Isopropylmethylketon in 65 ml Ethanol und 2,6 ml 97% iger Schwefelsäure eingesetzt wird.
Der resultierende Feststoff wird geschmolzen und mittels Kugelrohrdestillation im Vakuum (0,02-0,07 mbar; 105-110 °C) gereinigt. Ausbeute (Zwischenprodukt): 3,21 g (76 % der Theorie) 5-Fluoro-2,3,3-trimethyl-3H-indol
1,0 g des so erhaltenen Zwischenproduktes wird gemäss Variante 1 in 1b) mit 0,91 g Methyljodid und 3 ml Chloroform alkyliert.
Ausbeute: 0,70 g (39 % der Theorie),
¹H-NMR (CD₃OD: δ = 1,62 ppm (s, 6H); 4,06 ppm (s, 3H); 4,85 ppm (s, 3H); 7,38-7,42 ppm (m, 1H); 7,61-7,63 ppm (m, 1H) 7,86-7,89 ppm (m, 1H).
FAB-Massenspektrum: M⁺ = 192,20 (100% rel. Int.)

| Elementaranalyse: C₁₂H₁₅NFI (319,16) | | | | |
|---|---|---|---|---|
| | %C | %H | %N | %F |
| ber. | 45,16 | 4,74 | 4,39 | 5,95 |
| gef. | 44,70 | 4,90 | 4,30 | 6,00 |

### Beispiel 1.8: Synthese von 5-Isopropyl-1,2,3,3-tetramethyl-3H-indolium-jodid

Die Herstellung erfolgt gemäss der allgemeinen Synthesevorschrift 1a), wobei 3,71 g 4-lsopropylphenylhydrazin und 2,66 g Isopropylmethylketon in 90 ml Ethanol und 2.9 ml 97 %iger Schwefelsäure eingesetzt wird.
Das resultierende Oel wird durch Kugelrohrdestillation im Vakuum (0,02-0,07 mbar; 105-110 °C) gereinigt.
Ausbeute (Zwischenprodukt): 4,11 g (83 % der Theorie) 5-lsopropyl-2,3,3-trimethyl-3H-indol
1,0 g des so erhaltenen Zwischenproduktes wird gemäss Variante 1 in 1b) mit 0,81 g Methyljodid und 3 ml Chloroform alkyliert.
Ausbeute: 0,63 g (37 % der Theorie) 5-Isopropyl-1,2,3,3-tetramethyl-3H-indolium-jodid
¹H-NMR (CD₃OD): δ = 1.31 ppm (d, ³J = 7 Hz, 6H); 1,60 ppm (s, 6H); 3,06-3,12 ppm (m, 1H); 4,04 ppm (s, 3H); 4,84 ppm (s, 3H); 7,52 ppm (dd, ³J = 9 Hz, ⁴J= 2 Hz, 1H); 7,66 ppm (d, ⁴J = 2 Hz, 1H); 7,73 ppm (d, ³J = 8 Hz, 1H).
FAB-Massenspektrum: M⁺ = 216,3 (100% rel. Int.)

| Elementaranalyse: C₁₅H₂₂NJ (343,31) | | | |
|---|---|---|---|
| | %C | %H | %N |
| ber. | 52,48 | 6,46 | 4,08 |
| gef. | 52,90 | 6,80 | 3,82 |

### Beispiel 1.9: Synthese von 5-Hydroxy-1,2,3,3-tetramethyt-3H-indolium-jodid

Die Herstellung erfolgt gemäss der allgemeinen Synthesevorschrift 1c), wobei 4,0 g 5-Methoxy-2,3,3-trimethyl-3H-indol mit 16 ml Essigsäure und 16 ml 48%iger Bromwasserstofflösung eingesetzt wird.
Ausbeute (Zwischenprodukt): 2,45 g (66 % der Theorie) 5-Hydroxy-2,3,3-trimethyl-3H-indol
1,0 g des so erhaltenen Zwischenproduktes wird gemäss Variante 3 in 1b) mit 1,66 g Methyljodid und 10 ml Methanol alkyliert.
Ausbeute: 1,36 g (75 % der Theorie) 5-Hydroxy-1,2,3,3-tetramethyl-3H-indolium-jodid
Schmelzpunkt: 245-247°C
¹H-NMR (D₆-DMSO): δ = 1,46 ppm (s, 6H); 2,66 ppm (s, 3H); 3,89 ppm (s, 3H); 6,93 ppm (dd, ³J = 9 Hz , ⁴J = 2 Hz, 1H); 7,11 ppm (d, ⁴J = 2 Hz, 1H); 7,67 ppm (d, ³J = 9 Hz , 1H), 10,22 ppm (s, tauscht mit D₂O aus,1H) FAB-Massenspektrum: M⁺ = 190,10 (100% rel. lnt.)

| Elementaranalyse: C₁₂H₁₆NOJ (317,17) | | | | |
|---|---|---|---|---|
| | %C | %H | %N | %O |
| ber. | 45,44 | 5,08 | 4,42 | 5,04 |
| gef. | 45,50 | 5,60 | 4,40 | 5,60 |

### Beispiel 1.10: Synthese von 5-Nitro-1,3,3-trimethyl-2-methylenindolin

Die Herstellung erfolgt gemäss der allgemeinen Synthesevorschrift 1d), wobei 20 g 1,3,3-Trimethyl-2-methylenindolin gelöst in 50 ml 97 %iger Schwefelsäure 23,5 ml Nitriersäure (ein Gemisch bestehend aus 3,5 ml rauchender 99%iger Salpetersäure und 20 ml 97 %iger Schwefelsäure) verwendet werden. Das Rohprodukt wird in Hexan/Methylenchlorid umkristallisiert.
Ausbeute: 8,8 g (35 % der Theorie) 5-Nitro-1,3,3-trimethyl-2-methylen-indolin
Schmelzpunkt: 89 - 91 °C.
¹H-NMR (CDCl₃): δ = 1,35 ppm (s, 6H); 3.11 (s, 3H); 4,09 ppm (d, ²J = 3 Hz, 1 ); 4,11 ppm (d, ²J = 3 Hz, 1H); 6,51 ppm (d, ³J = 9 Hz,1H); 7,91 ppm (d, ⁴J = 2,5 Hz, 1H) 8,11 ppm (dd, ³J = 9 Hz, ⁴J = 2,5 Hz, 1H)
EI-Massenspektrum: 218 (85, M⁺); 203 (100); 188 (6); 171 (16); 157 (91); 145 (32); 128 (24); 115 (44); 103 (10); 89 (15); 77 (14); 63 (12)

### Beispiel 1.11: Synthese von 5-Methoxy-6-nitro-1,2,3,3-tetramethyl-3H-indolium-chlorid

Die Herstellung erfolgt gemäss der allgemeinen Synthesevorschrift 1d), wobei 0.50 g 5-Methoxy-1,2,3,3-tetramethyl-3H-indolium-iodid gelöst in 5 ml 97%iger Schwefelsäure verwendet wird. Man verwendet 0,5 ml Nitriersäure (Gemisch bestehend aus 1,5 ml rauchender, 99 %iger Salpetersäure und 10 ml 97%iger Schwefelsäure). Das Rohprodukt wird in ca. 2 ml heissem Acetonitril mit 3 M Salzsäure in Ethanol (ca. 0,5 ml) gelöst. Nach Zugabe von Wasser fällt das reine Produkt aus.
Ausbeute: 0,22 g (45 % der Theorie) 5-Methoxy-6-nitro-1,2,3,3-tetramethyl-3H-indolium-chlorid
¹H-NMR (D₆-DMSO): δ = 1,58 ppm (s, 6H); 2,78 ppm (s, 3H); 3,98 ppm (s, 3H); 4,05 ppm (s, 3H); 8,05 ppm (s, 1H); 8,60 ppm (s, 1H)
¹³C-NMR (D₆-DMSO): δ = 14,4 ppm (q): 21,5 ppm (q); 35,2 ppm (q); 54,7 ppm (s); 57,9 ppm (q); 110,2 ppm (d); 112,3 ppm (d); 134,5 ppm (s); 139,0 ppm (s); 147,4 ppm (s); 153,1 ppm (s); 196,2 (s).
FAB-Massenspektrum: M⁺ = 249,2 (100% rel. Int.)

| Elementaranalyse: C₁₃H₁₇N₂O₃Cl (284,74) | | | | | |
|---|---|---|---|---|---|
| | | %C | %H | %N | %O |
| | ber.: | 54,84 | 6,02 | 9,84 | 16,86 |
| | gef.: | 54,30 | 5,80 | 9,70 | 17,40 |

### Beispiel 1.12: Synthese von 5-N-Acetytamino-1,2,3,3-tetramethyl-3H-indolium-acetat

Die Herstellung erfolgt gemäss der allgemeinen Synthesevorschrift 1e), wobei für die Reaktion 1,3 g 5-Nitro-1,3,3-trimethyl-2-methylenindolin, 39 ml 32%ige Salzsäure und 8,10 g SnCl₂ x 2 H₂O eingesetzt werden.
Für die N-Acetylierungsreaktion verwendet man 1,48 g 5-Amino-1,2,3,3-tetramethyl-3H-indolium-chlorid, 37 ml Essigsäure und 37 ml Essigsäureanhydrid.
Ausbeute: 1,41 g (82 % der Theorie) 5-N-Acetylamino-1,2,3,3-tetramethyl-3H-indolium-acetat
¹H-NMR (D₆-DMSO): δ = 1,48 ppm (s, 6H); 1,90 (s, 3H aus AcO⁻); 2,09 ppm (s, 3H); 2,71 ppm (s, 3H); 3,93 ppm (s, 3H); 7,74 ppm (dd, ³J = 9 Hz, ⁴J = 2 Hz, 1H) 7,83 ppm (d, ³J = 9 Hz, 1H); 8,08 ppm (d, ⁴J = 2 Hz, 1H), 10,73 ppm(s, 1H).
EI-Massenspektrum: 230 (67, M⁺); 215 (100); 199 (5); 187 (10); 173 (25); 145 (35); 130 (10); 115 (6); 103 (5); 77 (8)

### Beispiel 1.13: Synthese von 1,2-Dimethyl-5-methoxy-3-(spirocyctohexyl)-3H-indolium-tetrafluoroborat

Die Herstellung erfolgt gemäss der allgemeinen Synthesevorschrift 1a), wobei 3,0 g 4-Methoxyphenylhydrazin und 3,42 g Cyclohexylmethylketon in 50 ml Ethanol und 4,43 g 97%iger Schwefelsäure eingesetzt werden. Das resultierende Oel wird mittels Chromatographie (Silicagel; Hexan:EtOAc = 6:4) gereinigt.
Es werden 1,46 g (29 % der Theorie) 5-Methoxy-2-methyl-3-(spirocyclohexyl)-3H-indol erhalten.
1,11 g des so erhaltenen Zwischenproduktes wird gemäss Synthesevorschrift 1b) (Variante 4) mit 3,10 g Trimethyloxonium-tetrafluoroborat (4,3 Äquivalente) und 20 ml 1,2-Dichlorethan alkyliert. Ausbeute: 1,26 g (79 % der Theorie) 1,2-Dimethyl-5-methoxy-3-(spirocyclohexyl)-3H-indolium-tetrafluoroborat
¹H-NMR (D₆-DMSO): 1,33-1,60 ppm (m, 3H); 1,70-2,10 ppm (m, 7H); 2,71 ppm (s, 3H); 3,88 ppm (s, 3H); 3,92 ppm (s, 3H); 7,21 ppm (dd, ³J = 9 Hz , ⁴J = 2,0 Hz, 1H), 7,51 ppm (d, ⁴J = 2,0 Hz, 1H); 7,86 ppm (d, ³J = 9,0 Hz, 1H);
FAB-Massenspektrum: M⁺ = 244,2 (100% rel. Intensität)

| Elementaranalyse: C₁₆H₂₂NOBF₄ (331,16) | | | | | |
|---|---|---|---|---|---|
| | %C | %H | %N | %F | %O |
| ber. | 58,03 | 6,70 | 4,23 | 22,95 | 4,83 |
| gef. | 57,70 | 6,80 | 4,20 | 23,00 | 4,80 |

### Beispiele 2.1 bis 5.5 : Haarfärbelösung

| **Indolhaltige Komponente A1** | |
|---|---|
| Indolderivat der Formel (I)/(Ia) | Mengenangaben gemäss Tabellen 1-4 |
| Laurylethersulfat (28 %ige wässrige Lösung) | 1 g |
| Ethanol | 2 g |
| Wasser, vollentsatzt | ad 10 g |

Der pH-Wert der Lösung wird mit 20%iger wässriger Monoethanolaminlösung auf den in den Tabellen 1 bis 4 genannten Wert eingestellt.

| **Aldehydhaltige Komponente A2** | |
|---|---|
| Aldehydverbindung | Mengenangaben gemäß Tabellen 1-4 |
| Laurylethersulfat (28 %ige wässrige Lösung) | 1 g |
| Ethanol | 2 g |
| Wasser, vollentsalzt | ad 10 g |

Der pH-Wert der Komponente A2 liegt zwischen 4 und 5.

1 g der Komponente A1 wird mit 1 g der Komponente A2 vermischt, wobei der resultierende pHₘ-Wert (pH-Wert der Mischung der Komponenten A1+A2) in den Tabellen 1 bis 4 angegeben ist.

Das erhaltene gebrauchsfertige Haarfärbemittel wird auf gebleichte Haare aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Das Haar kann zu einem beliebigen Zeitpunkt (beispielsweise nach mehreren Tagen oder Wochen) innerhalb von 20 Minuten bei 40 °C mit einer sauren (pH=5) 5 %igen Natriumsulfit-Lösung wieder vollständig entfärbt werden.

Die Färbe- und Entfärbeergebnisse sind in den nachfolgenden Tabellen 1 bis 4 zusammengefasst.

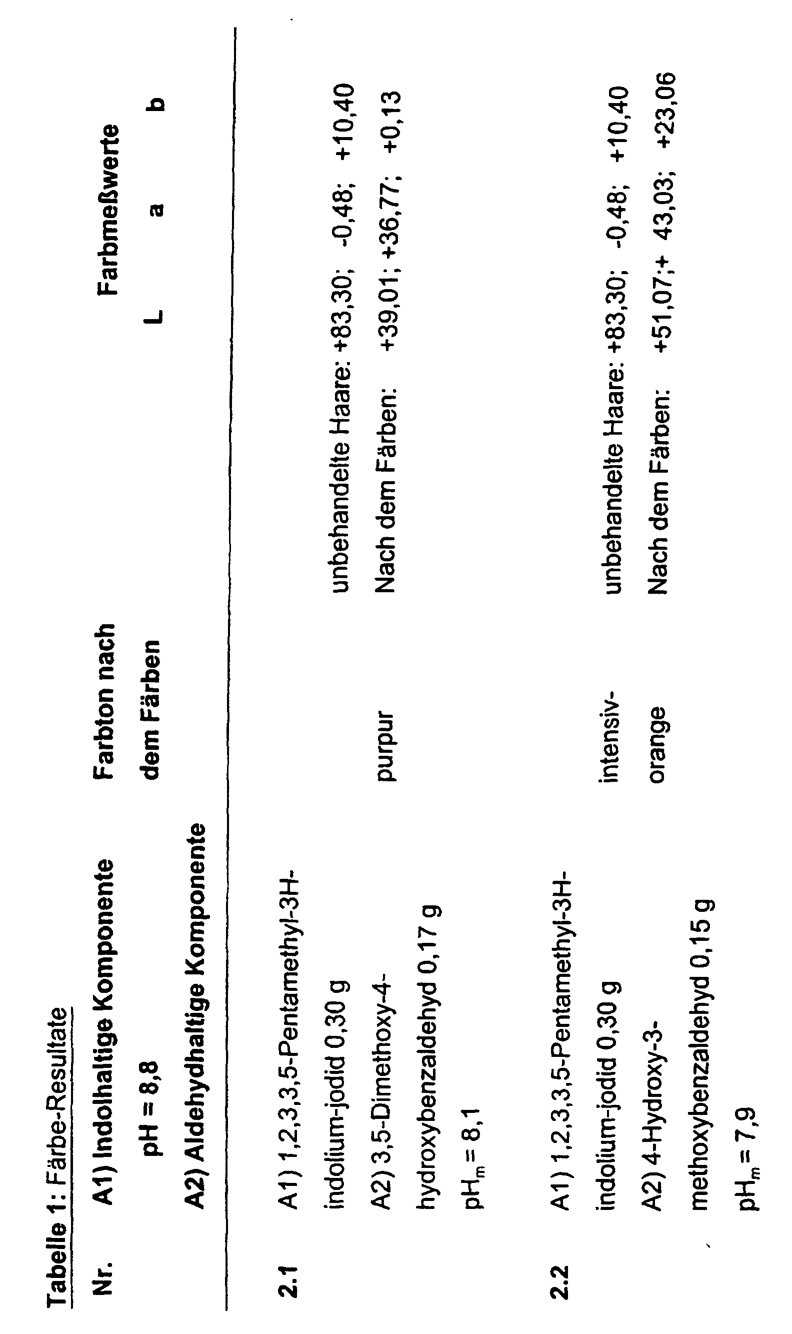

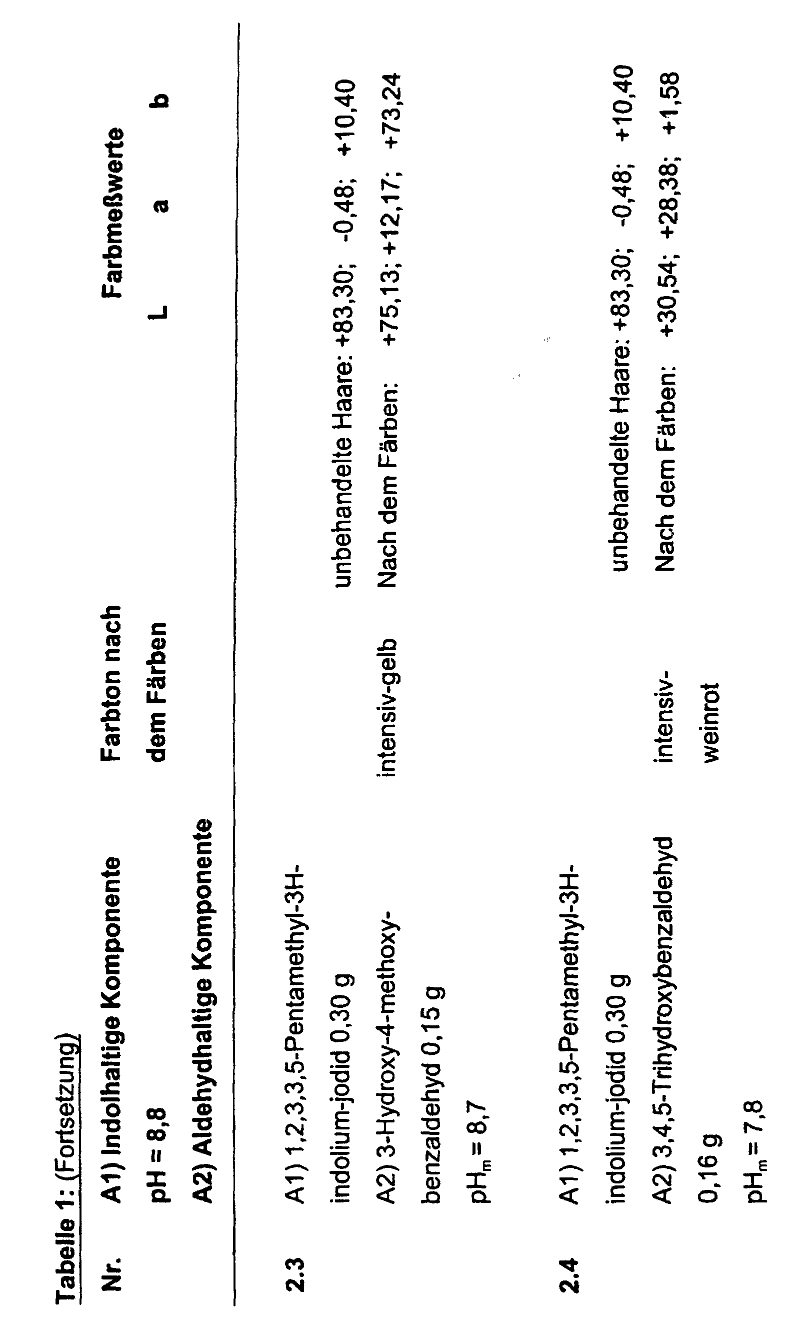

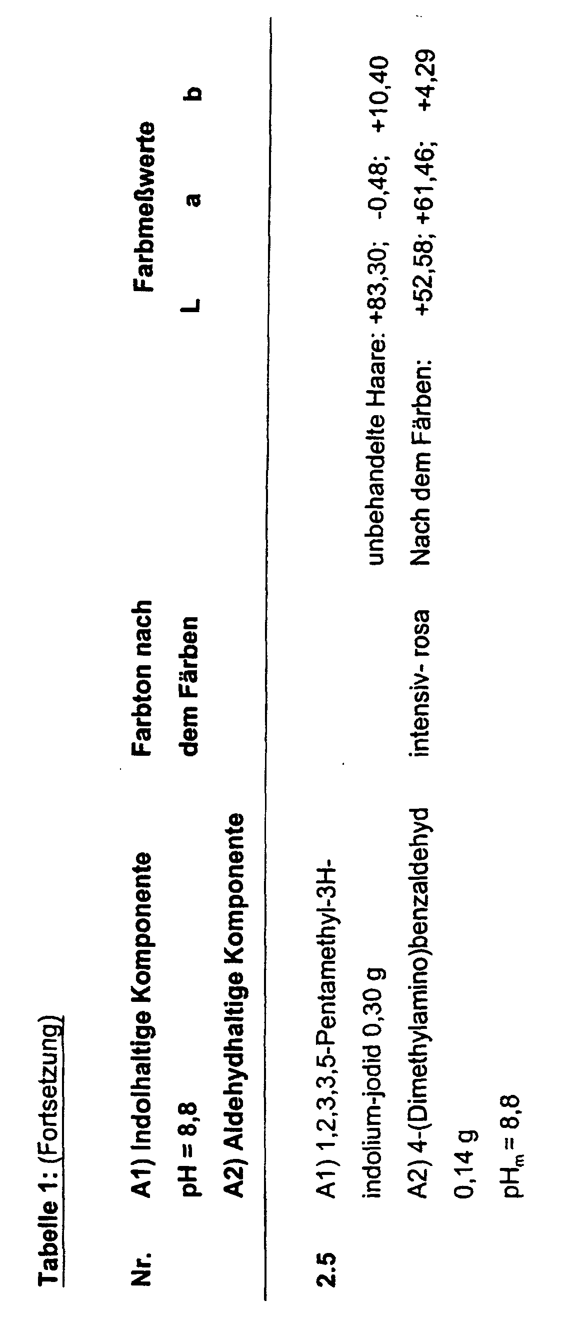

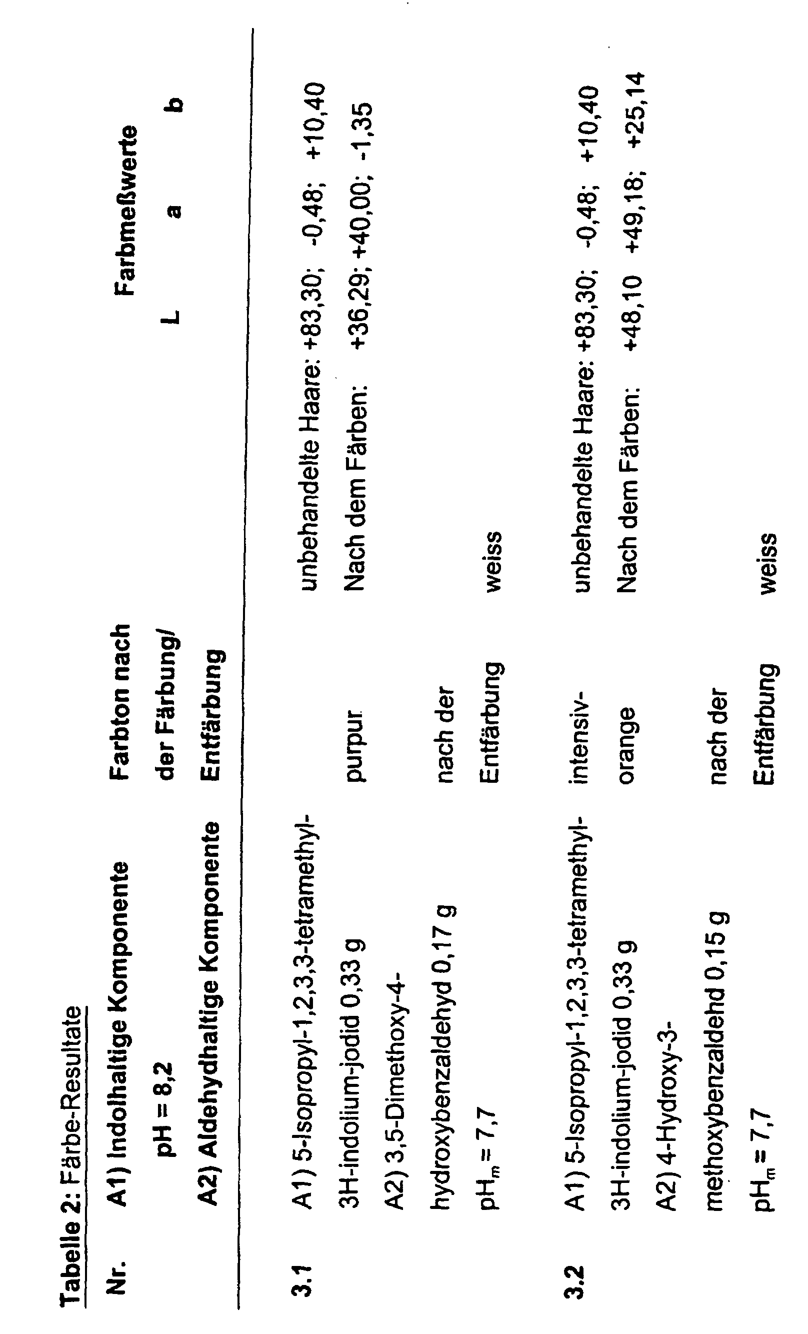

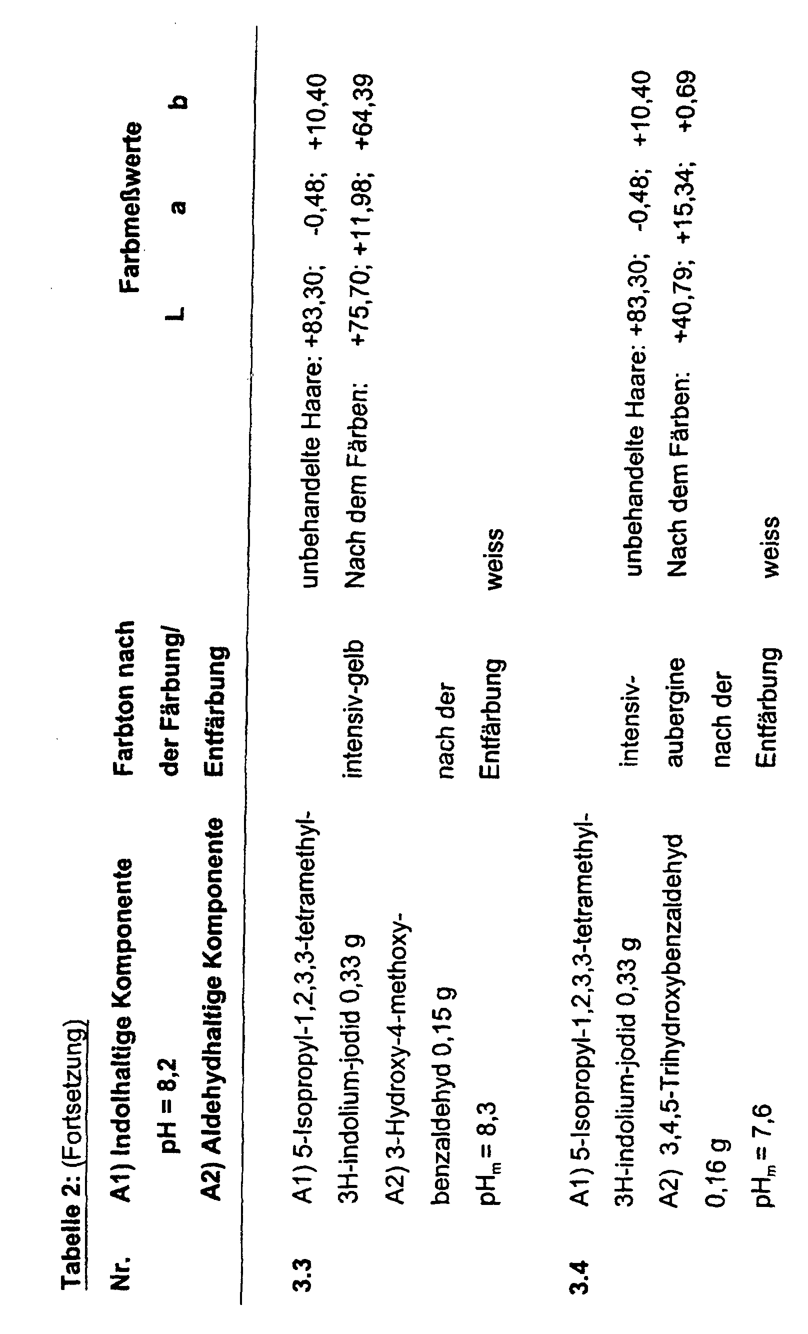

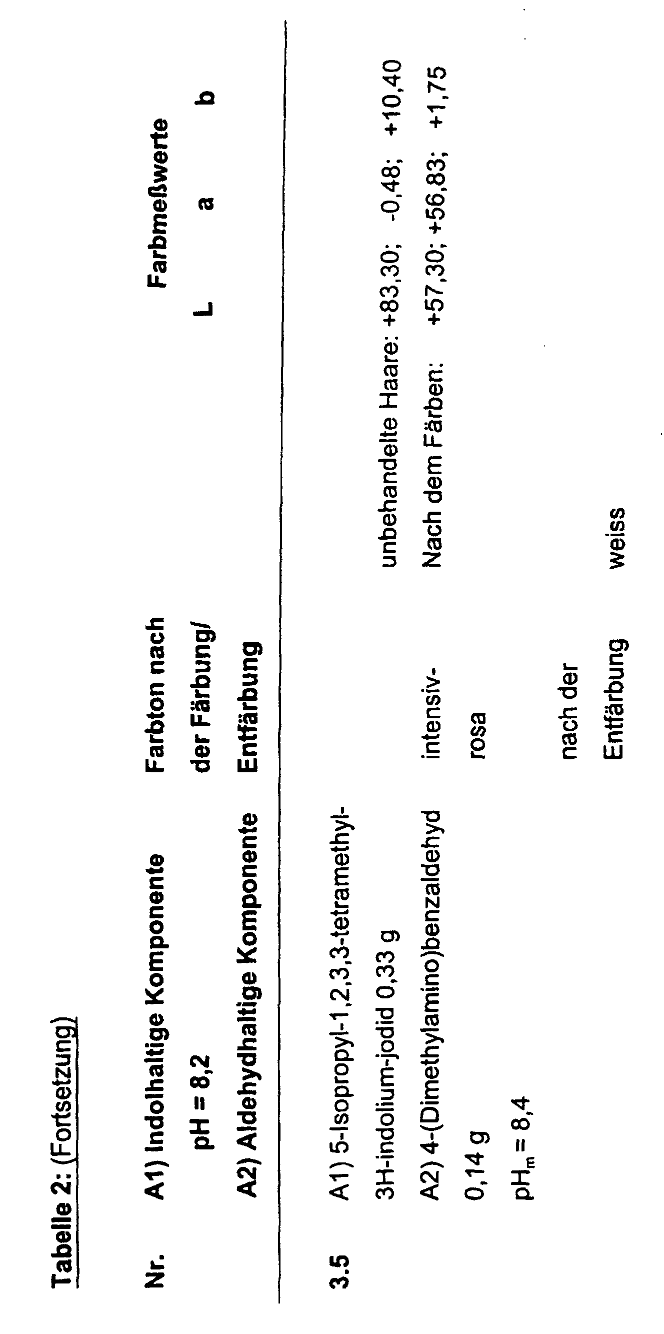

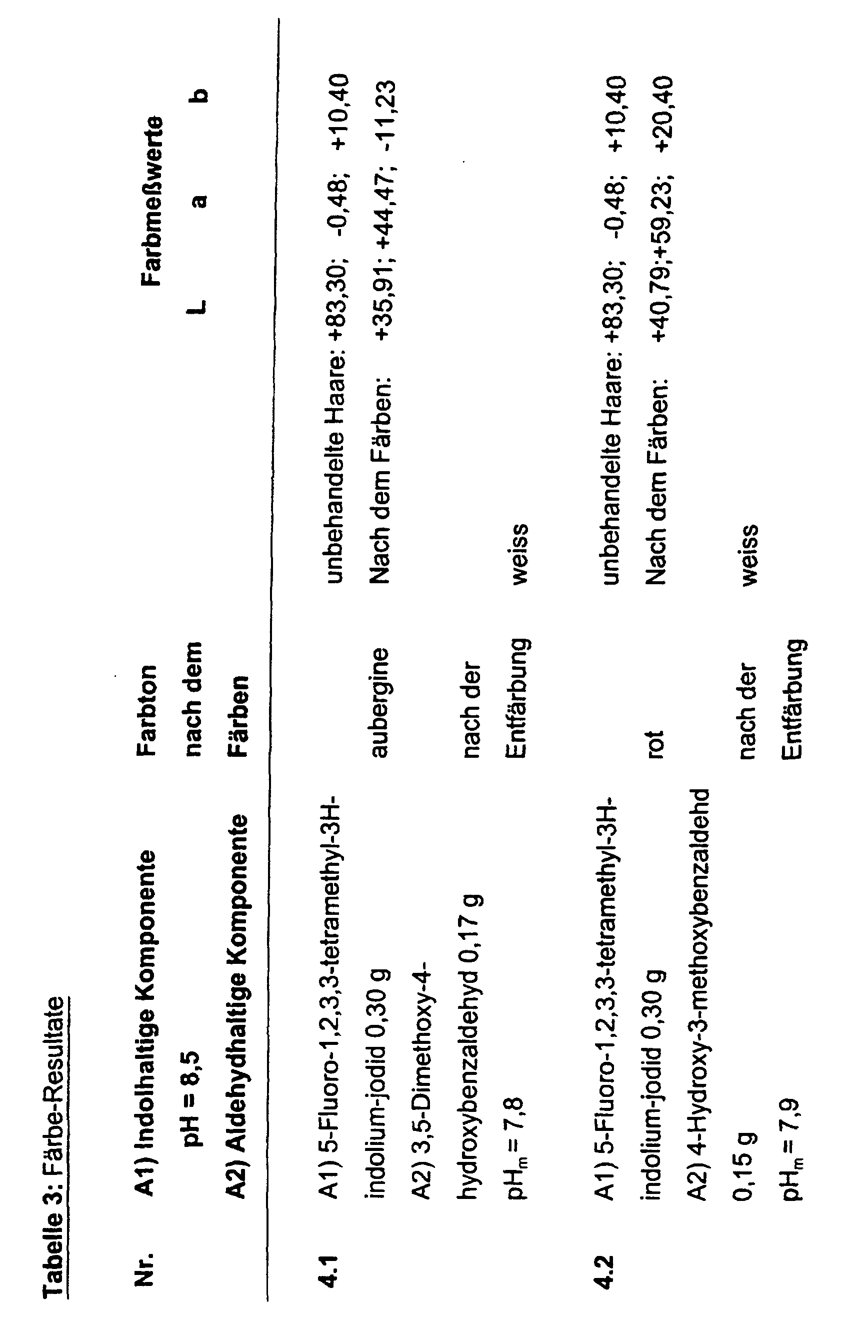

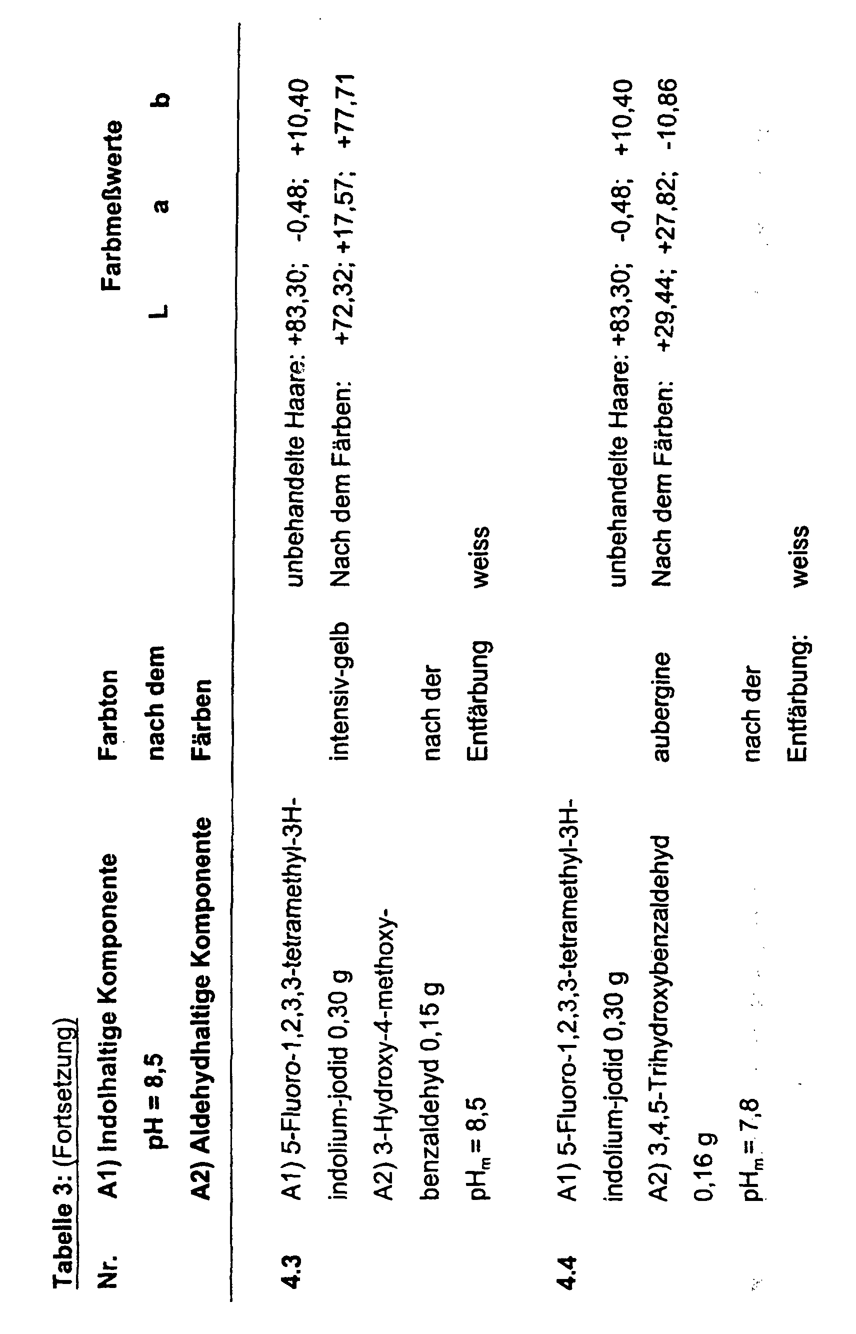

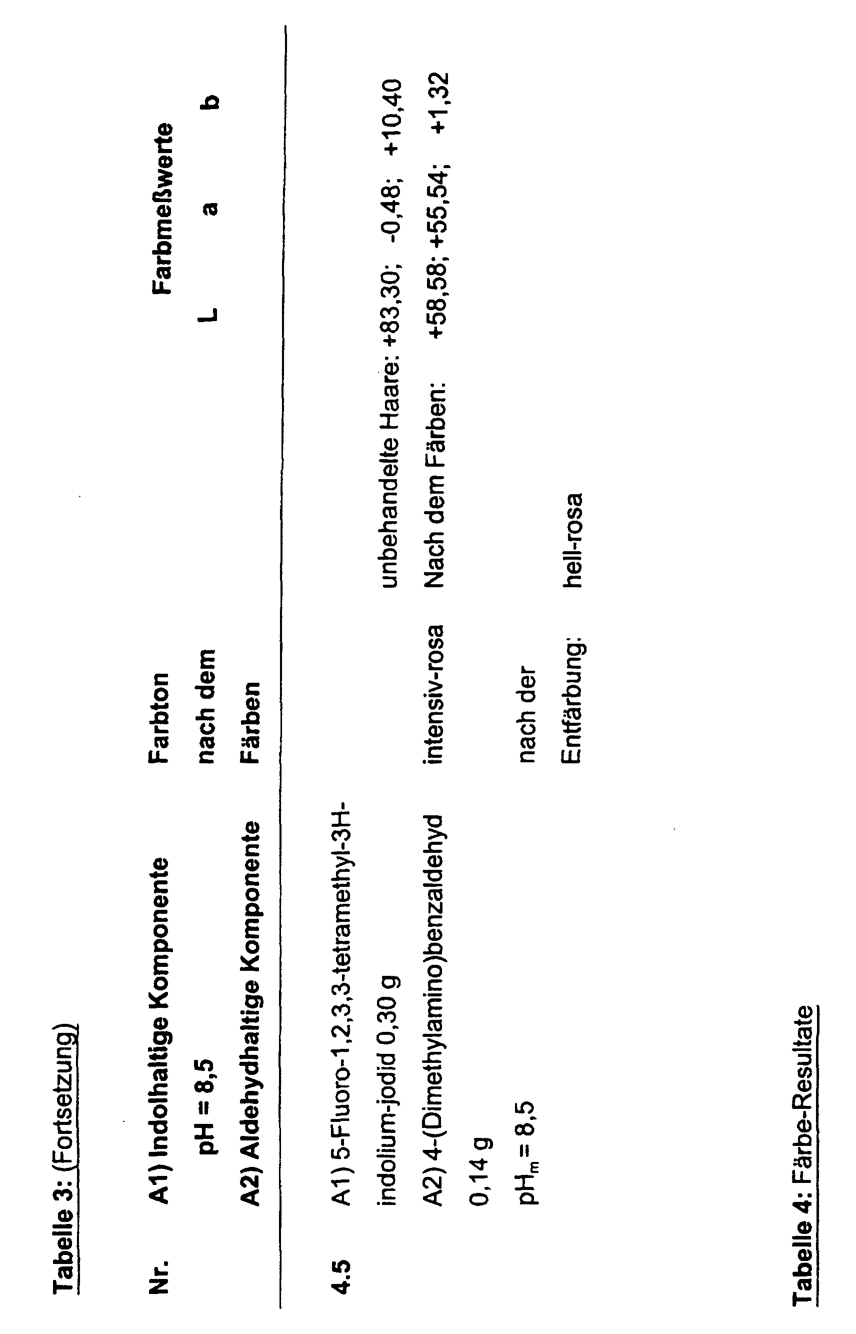

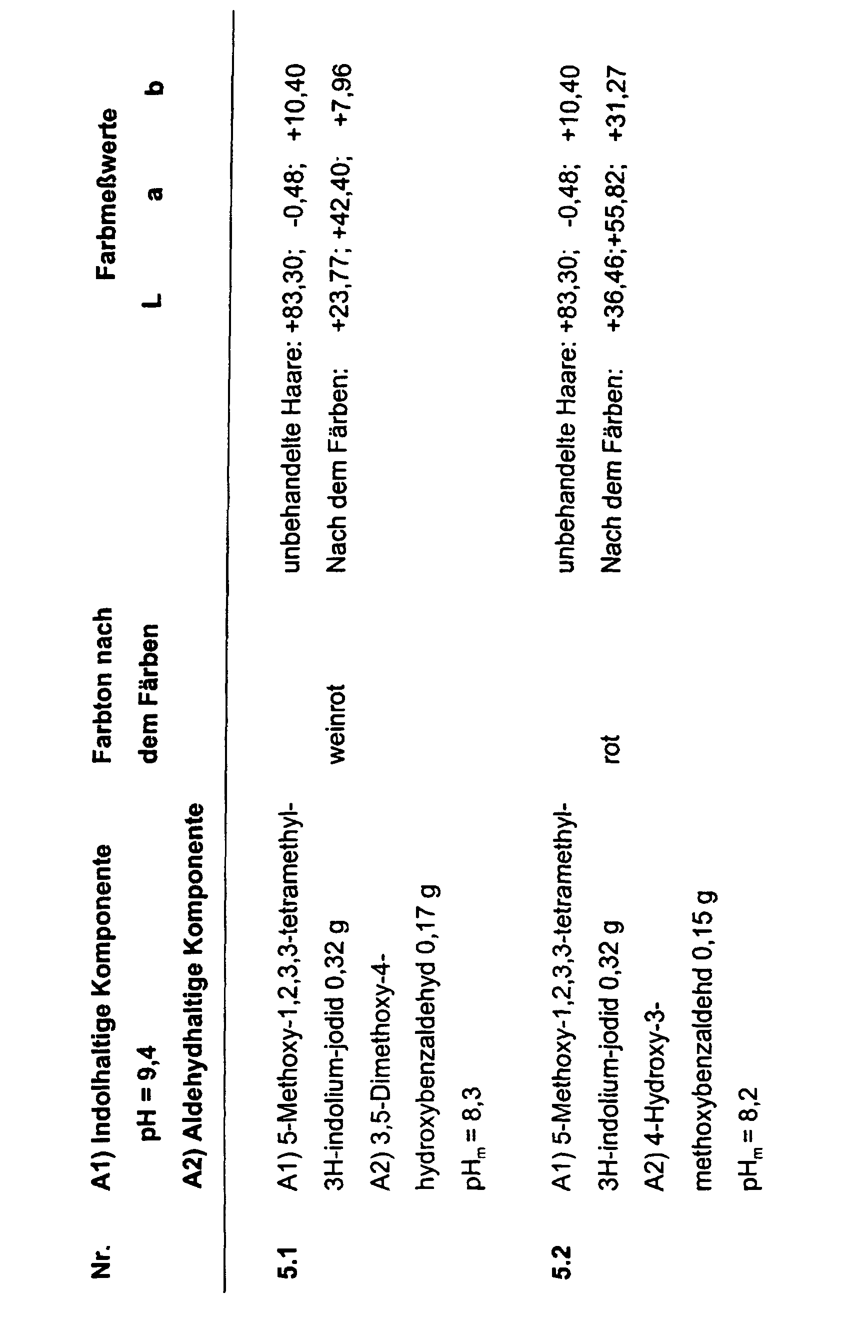

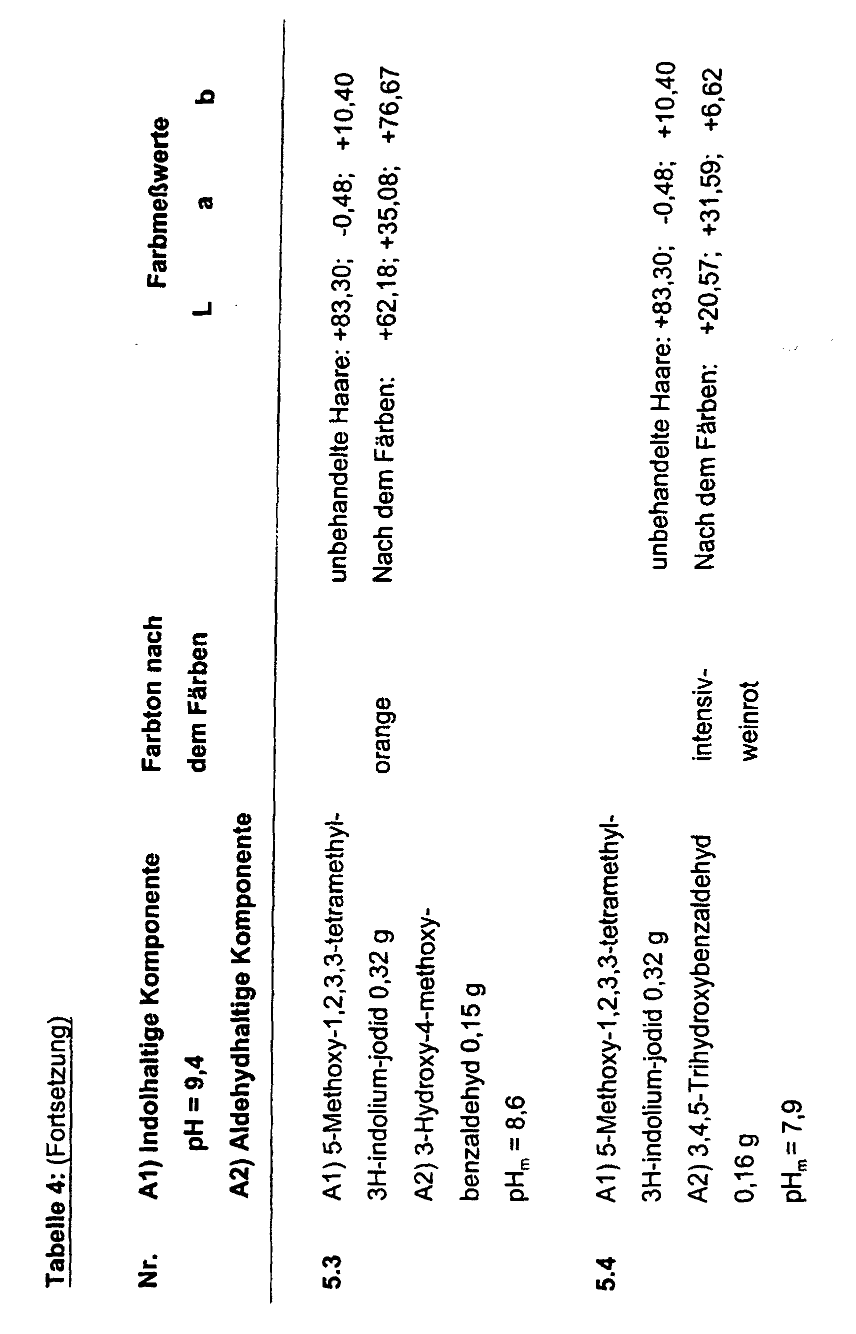

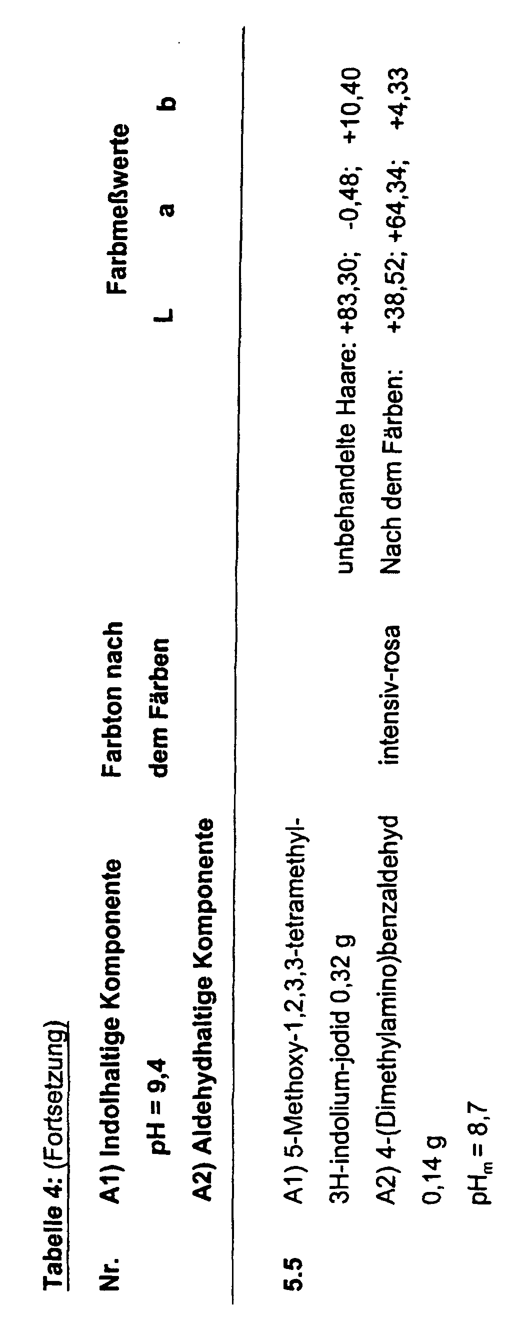

### Färbebeispiele 6.1 bis 12.5: Haarfärbemittel in Cremeform

| **Komponente A1 mit Indolderivat** | |
|---|---|
| Indolderivat der Formel (I)/(la) | Mengenangaben gemäss Tabellen 5-11 |
| Cetylstearylalkohol | 12 g |
| Laurylethersulfat, 28%ige wässrige Lösung | 10 g |
| Ethanol | 23 g |
| Wasser, vollentsalzt | ad 100 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird Verbindung (I)/(Ia), mit dem Ethanol und dem restlichen Wasser versetzt, zugegeben. Der pH-Wert der Creme wird mit 20%iger wässriger Monoethanolaminlösung auf den in den Tabellen 5 bis 10 angegebenen Wert eingestellt.

| **Aldehydhaltige Komponente A2** | |
|---|---|
| Aldehydverbindung | Mengenangaben gemäss Tabellen 5-10 |
| Cetylstearylalkohol | 12 g |
| Laurylethersulfat, 28 %ige wässrige Lösung | 10 g |
| Ethanol | 23 g |
| Wasser, vollentsalzt | ad 100 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird der Aldehyd zusammen mit dem Ethanol und dem restlichen Wasser zugegeben.
Der pH-Wert der Komponente A2 liegt zwischen 4 und 5.

Die Komponente A1 und die Komponente A2 werden im Verhältnis 1:1 miteinander vermischt. Der gemessene pH-Wert der Komponenentenmischung wird in den Tabellen 5 bis 11 als pHₘ bezeichnet. Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit einem Shampoo gewaschen, anschliessend mit lauwarmem Wasser gespült und sodann getrocknet.

Das Haar kann zu einem beliebigen Zeitpunkt (beispielsweise nach mehreren Tagen oder Wochen) innerhalb von 20 Minuten bei 40 °C mit einer sauren (pH=5) 5%igen Natriumsulfit-Lösung (Komponente B) wieder vollständig entfärbt werden.

Die Färbe- und Entfärbeergebnisse sind in den nachfolgenden Tabellen 5 bis 11 zusammengefasst.

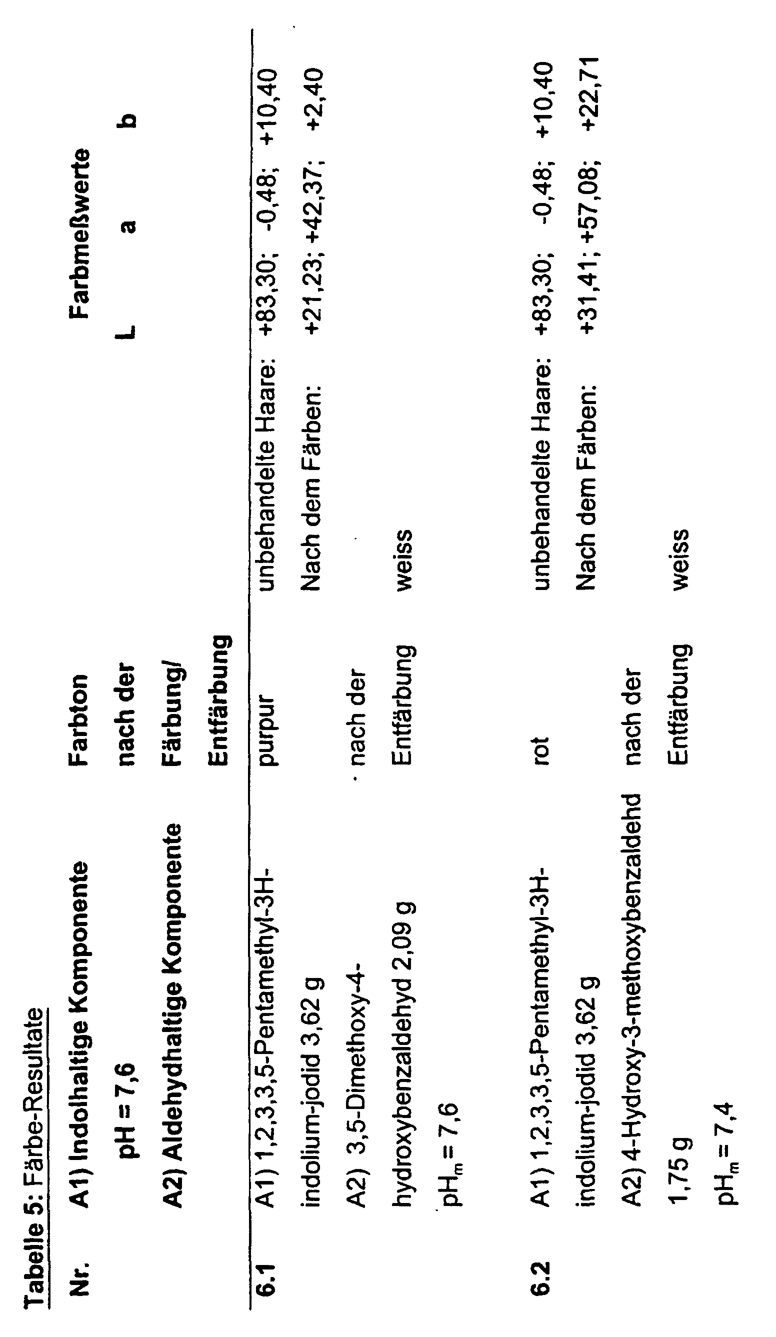

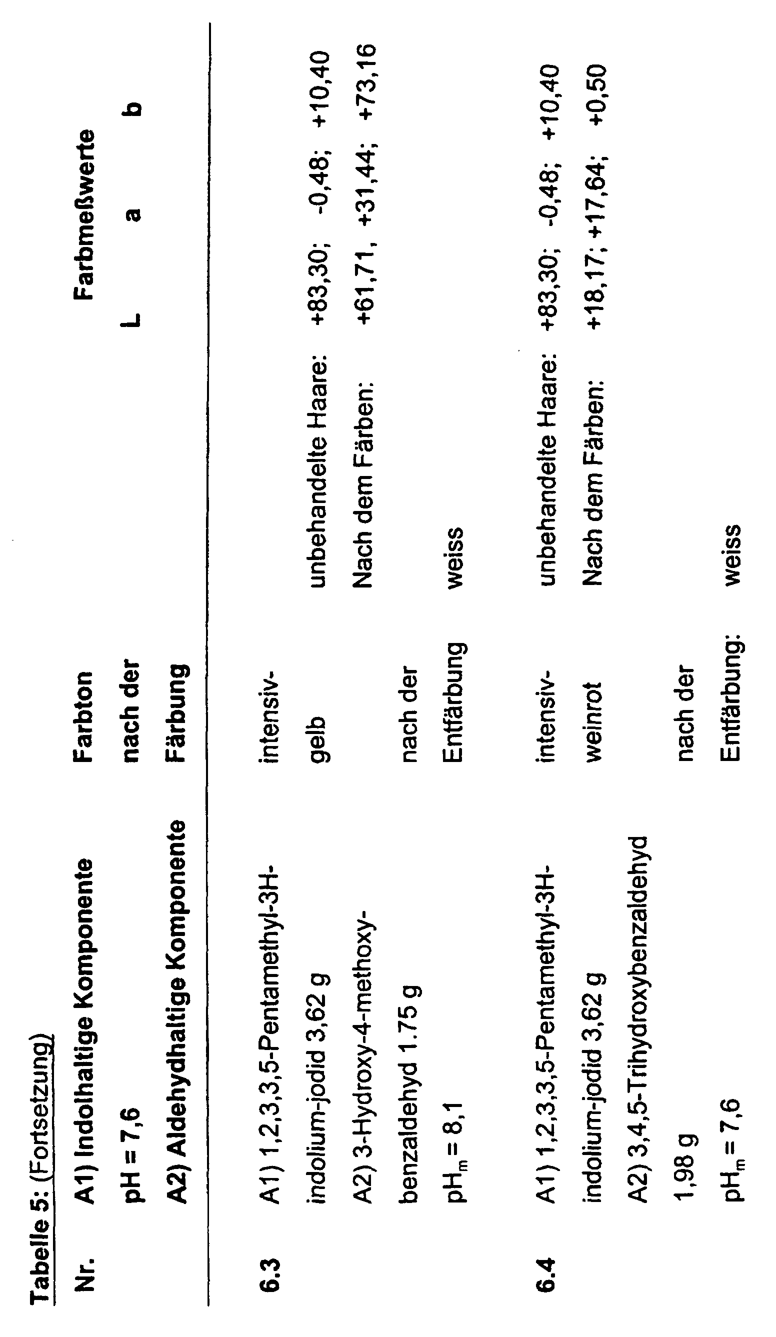

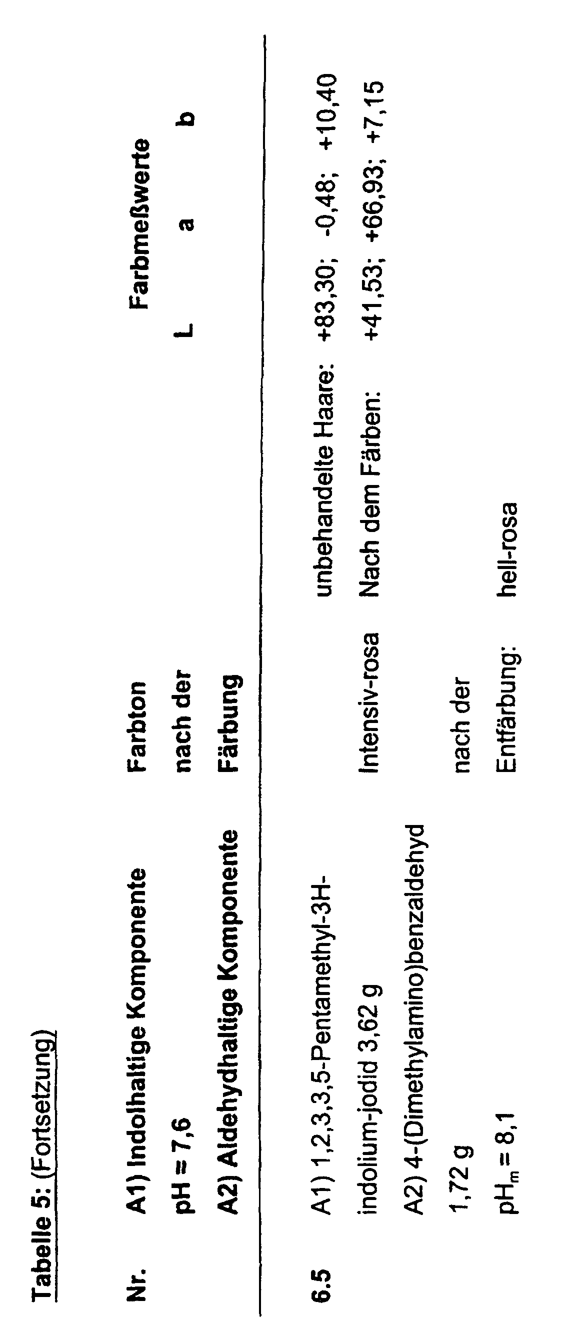

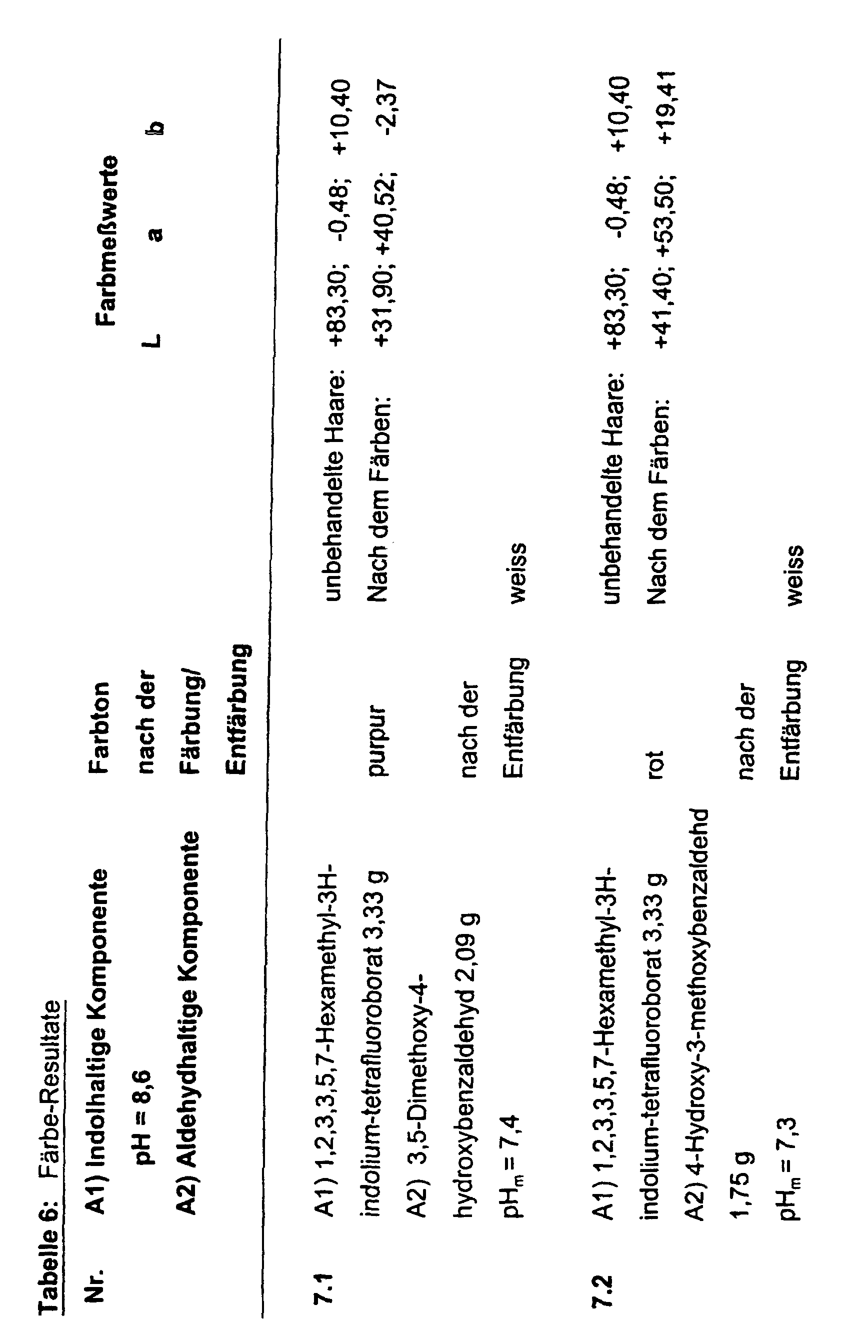

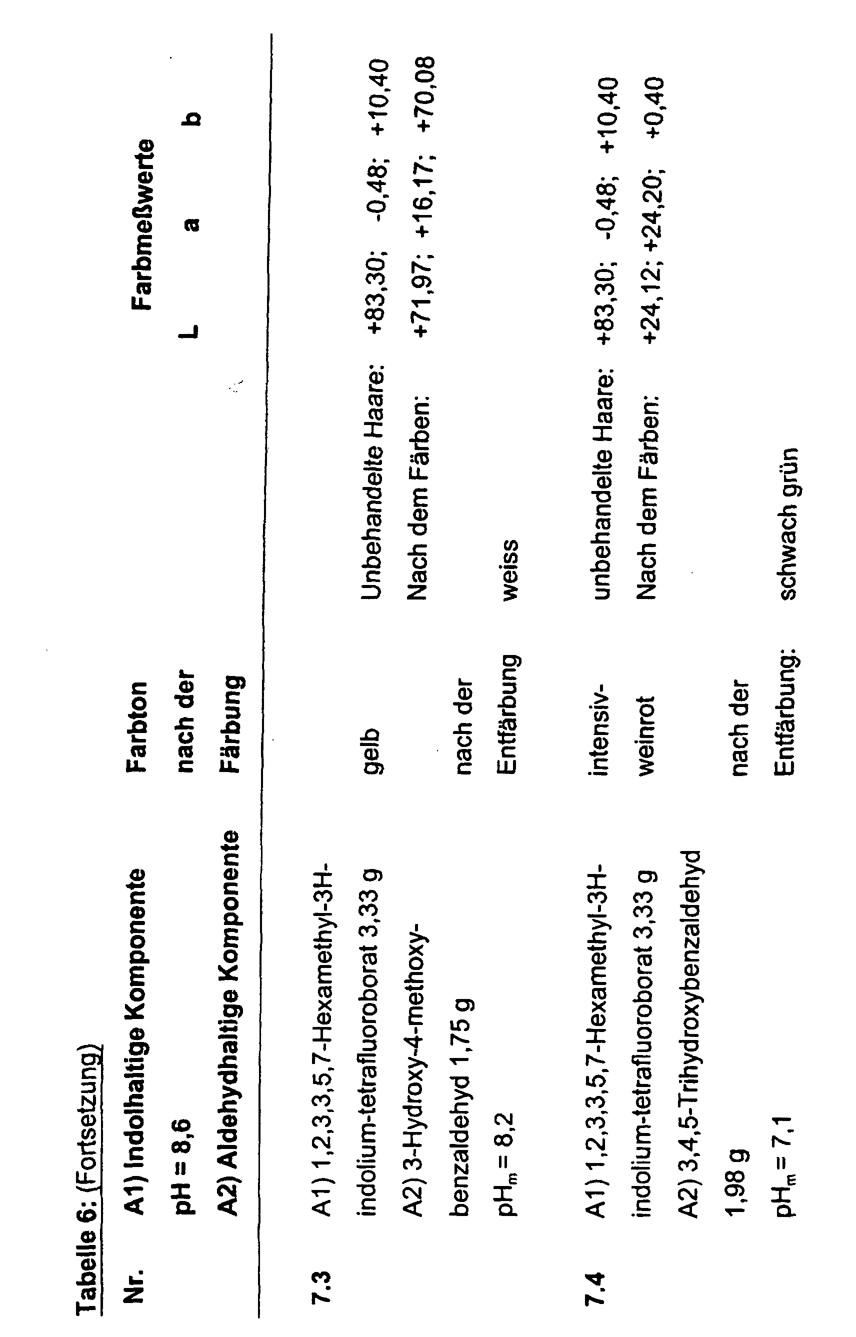

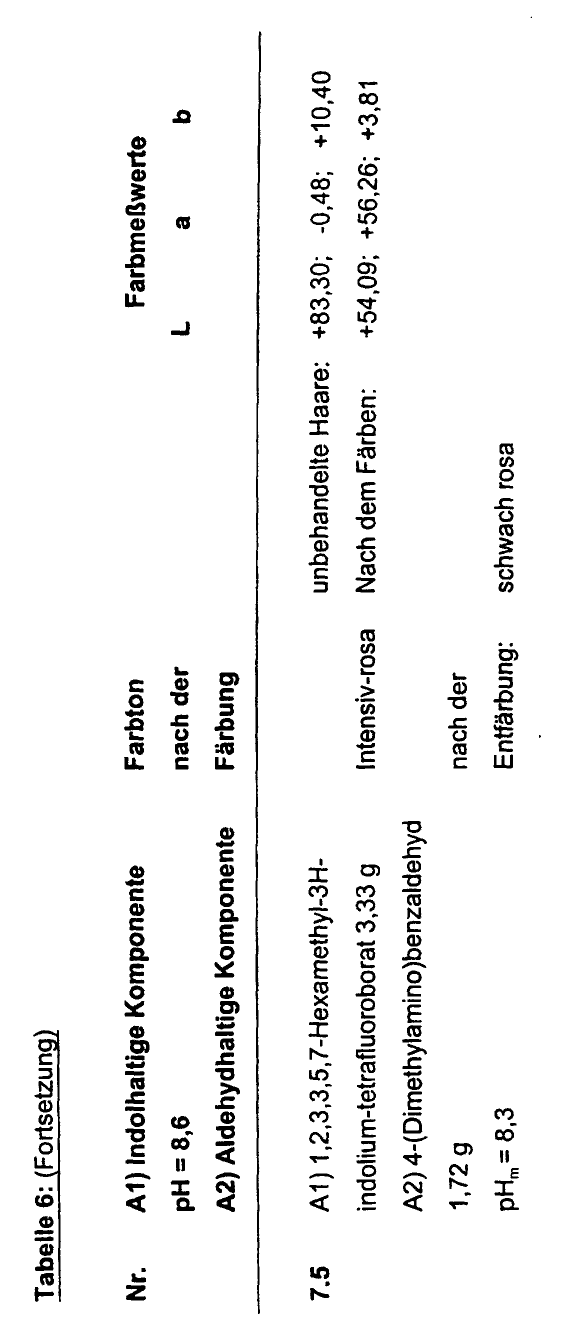

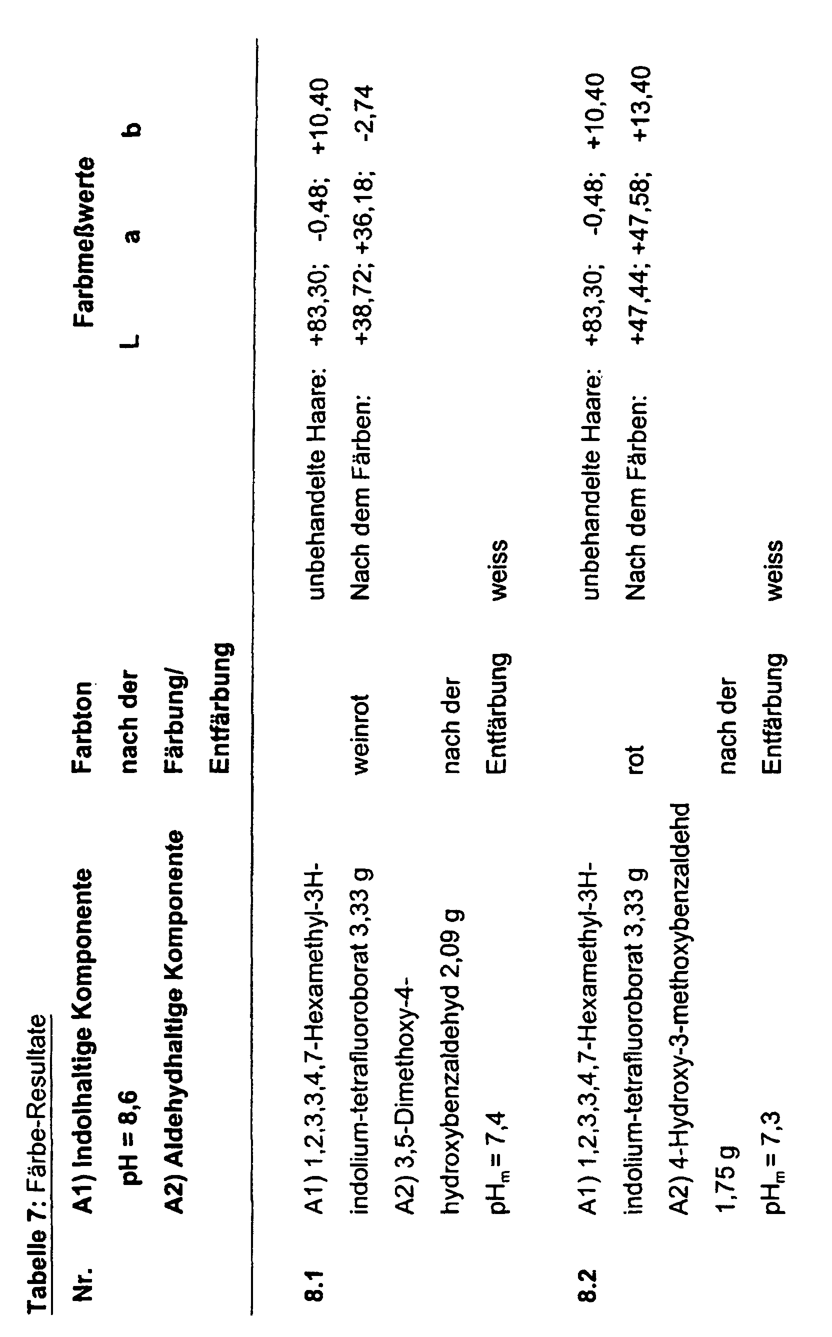

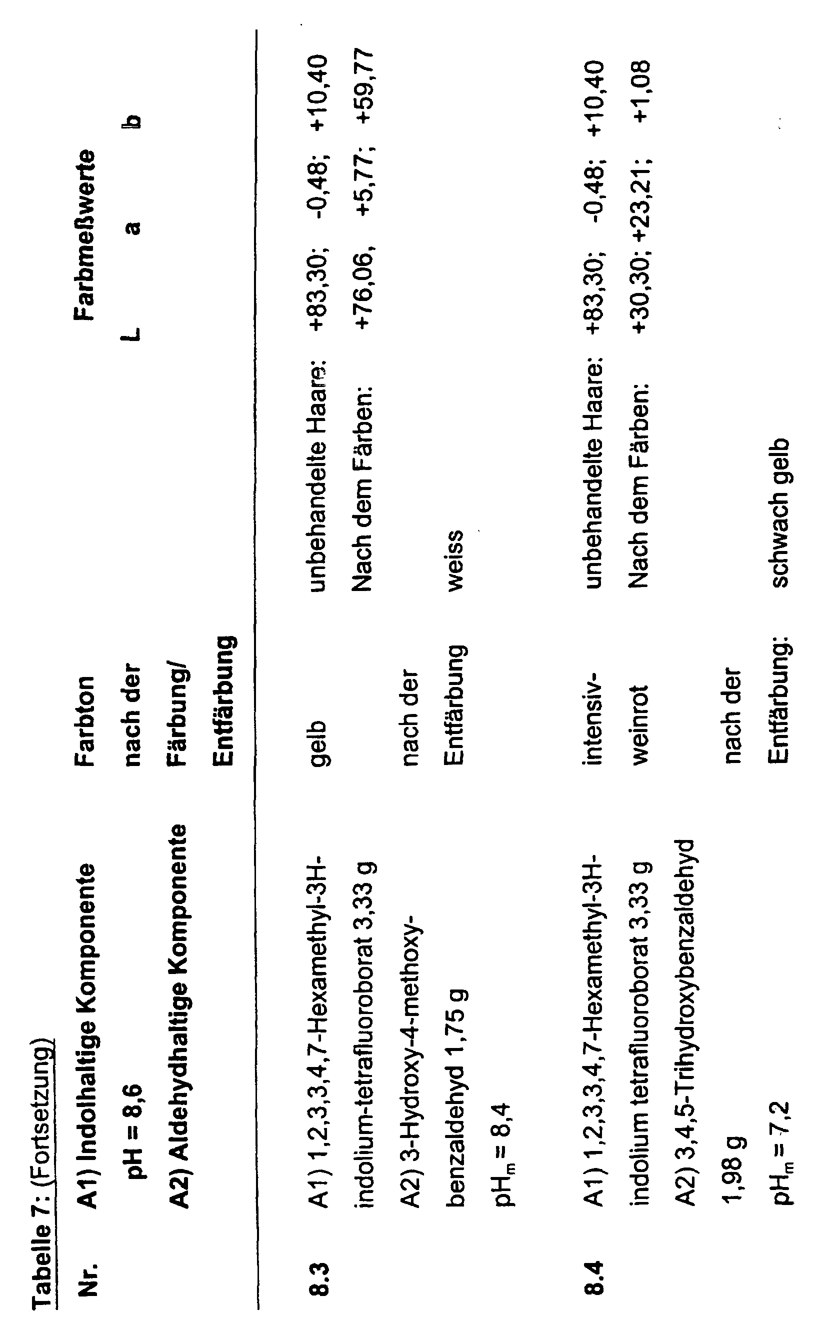

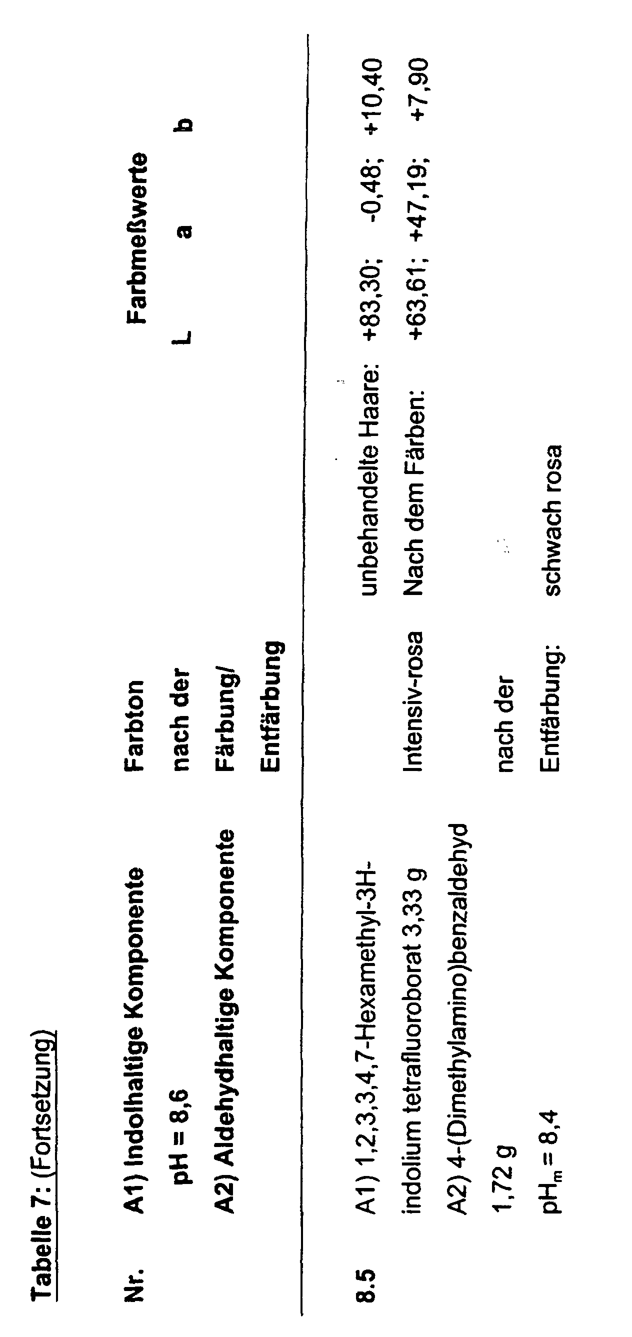

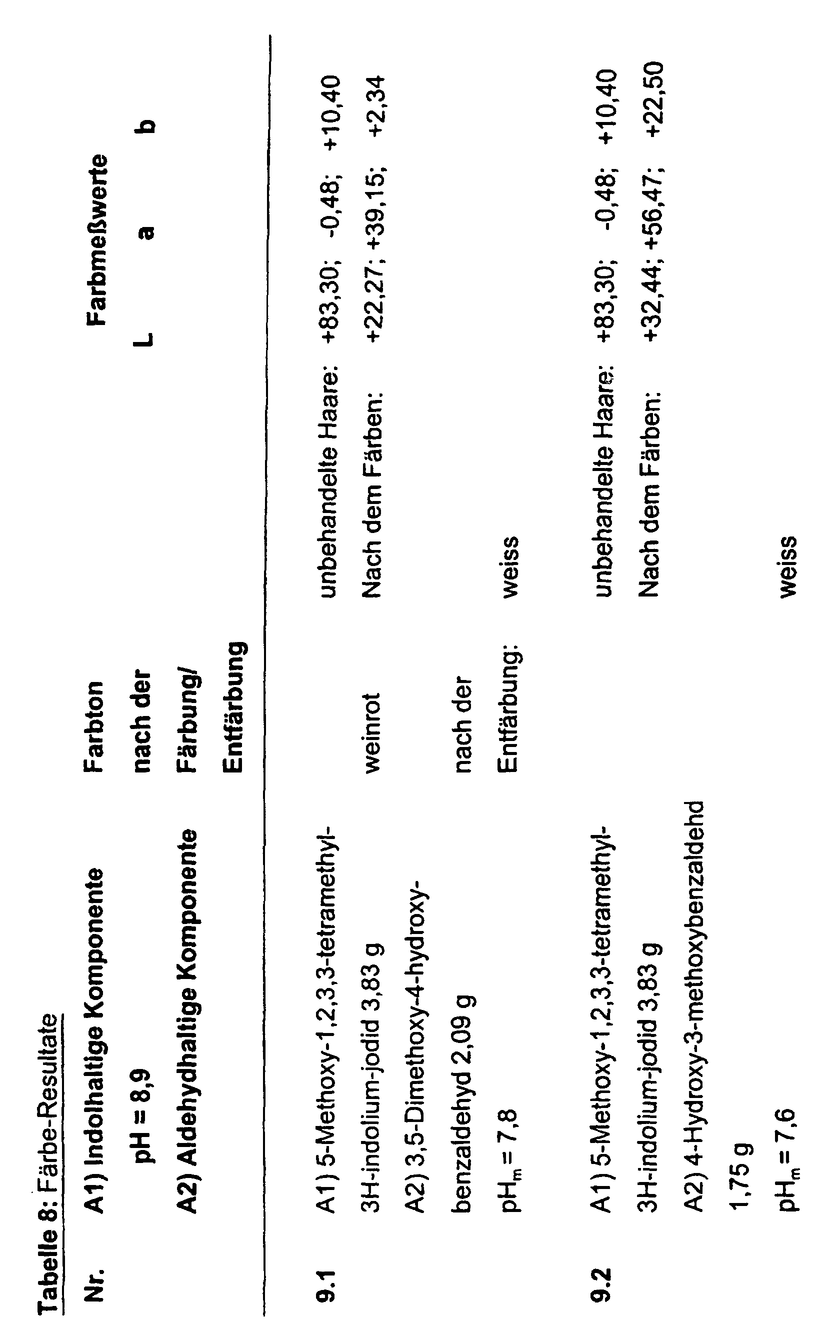

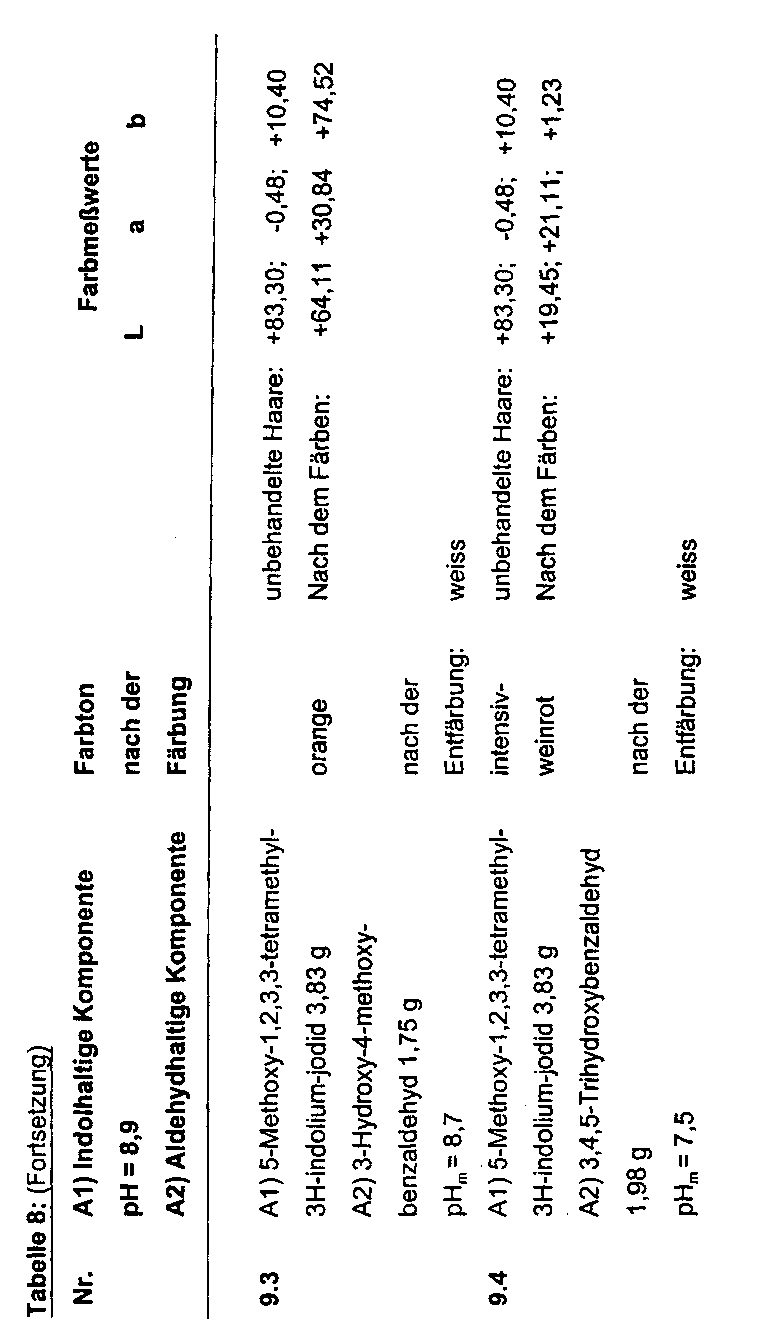

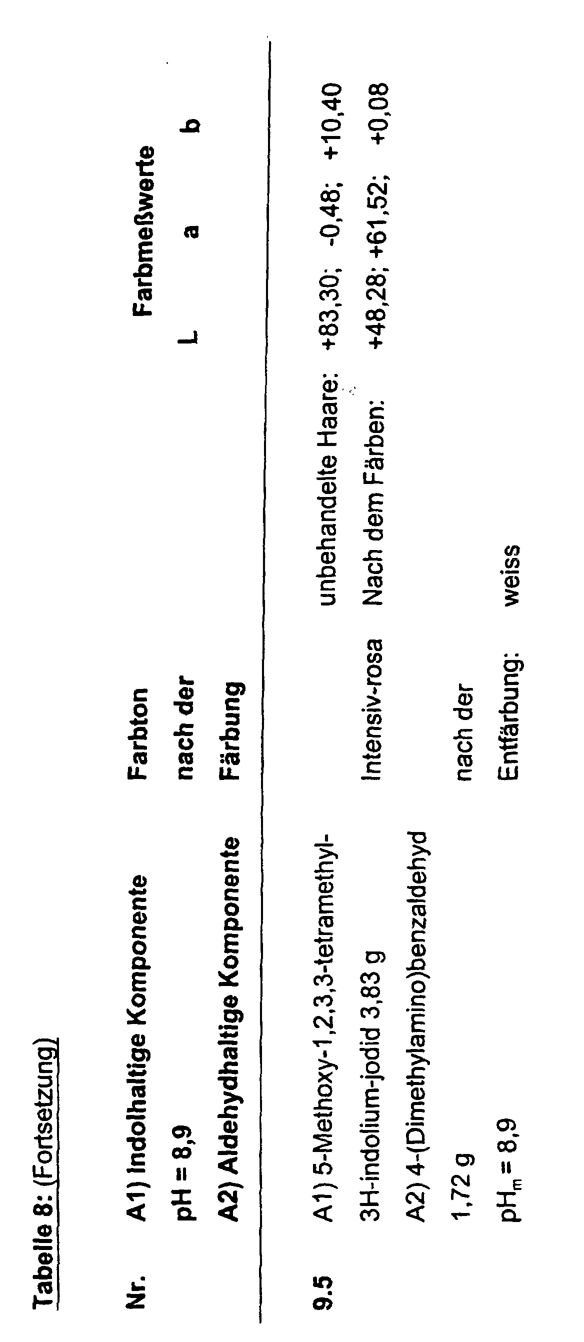

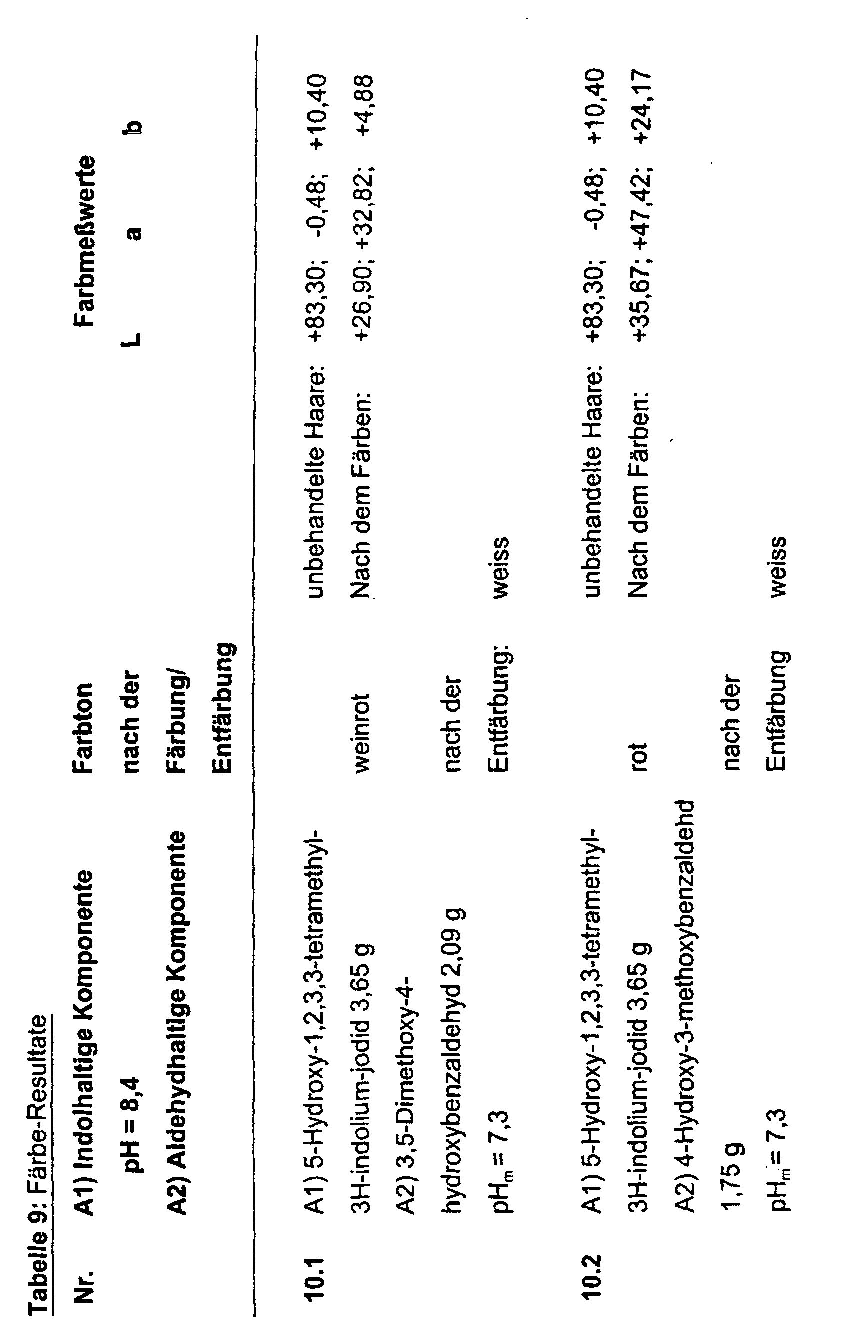

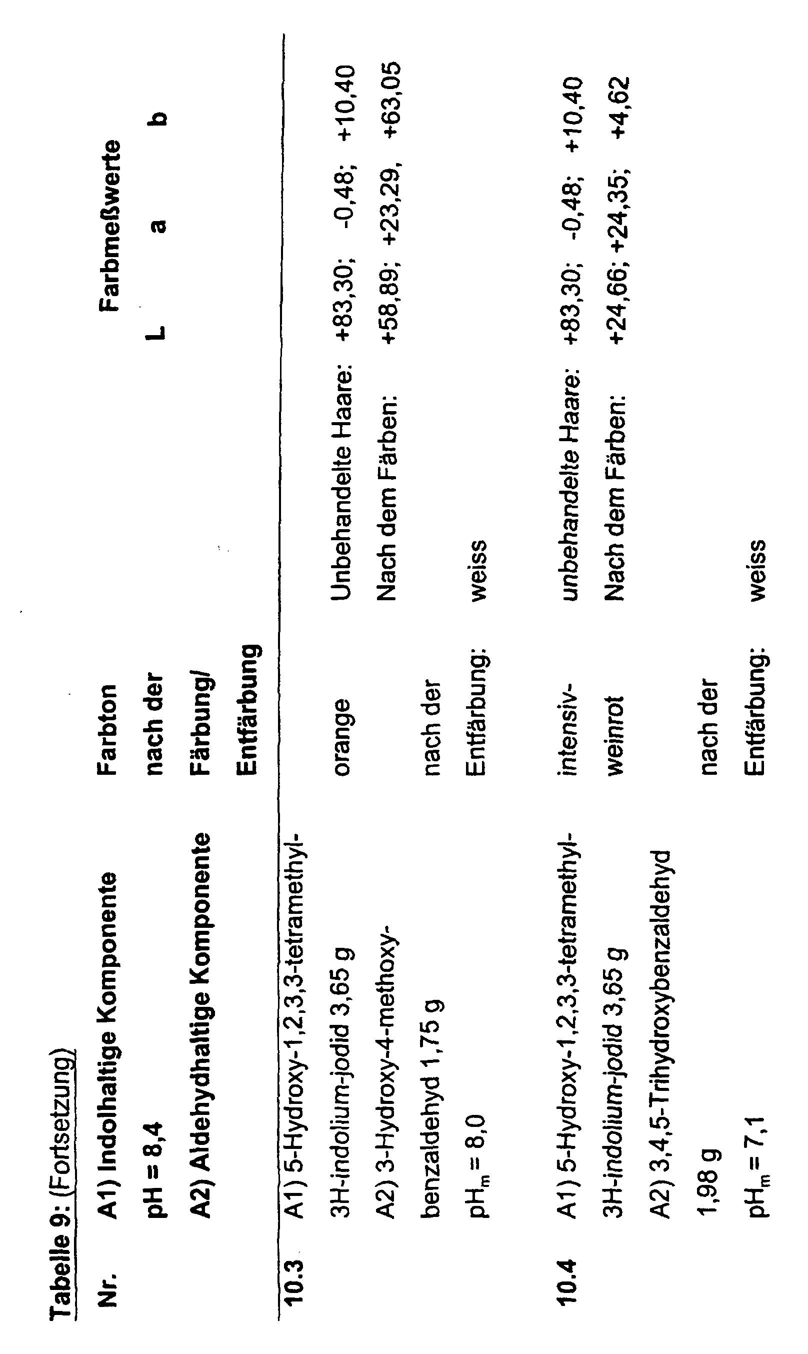

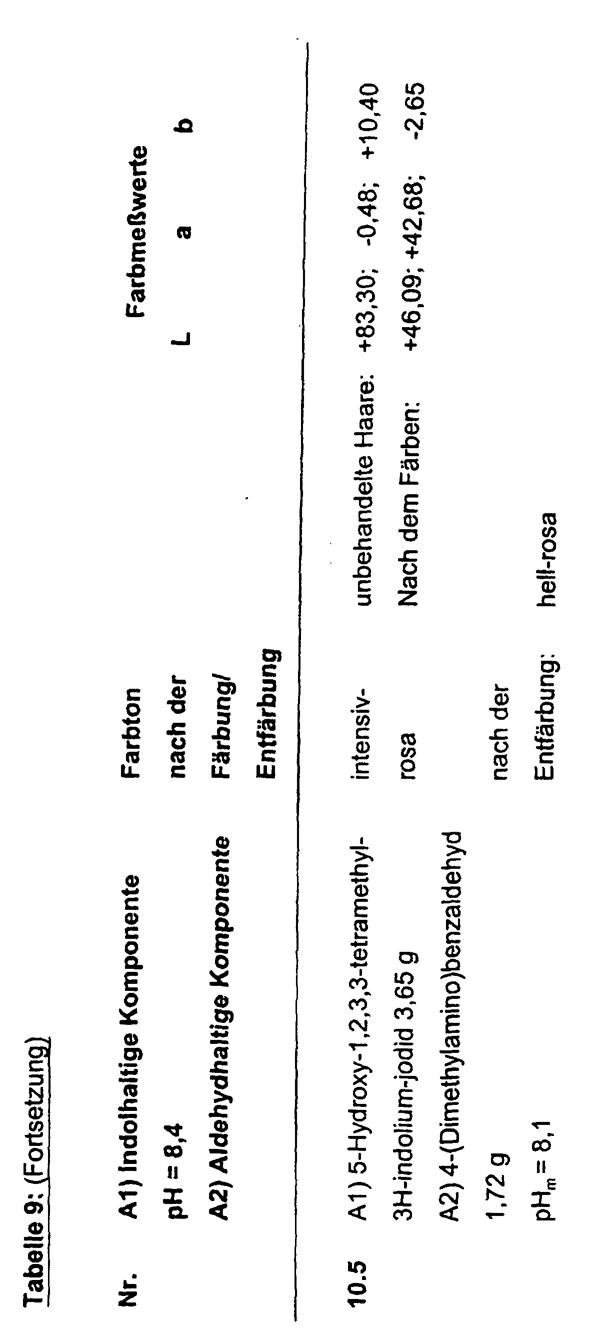

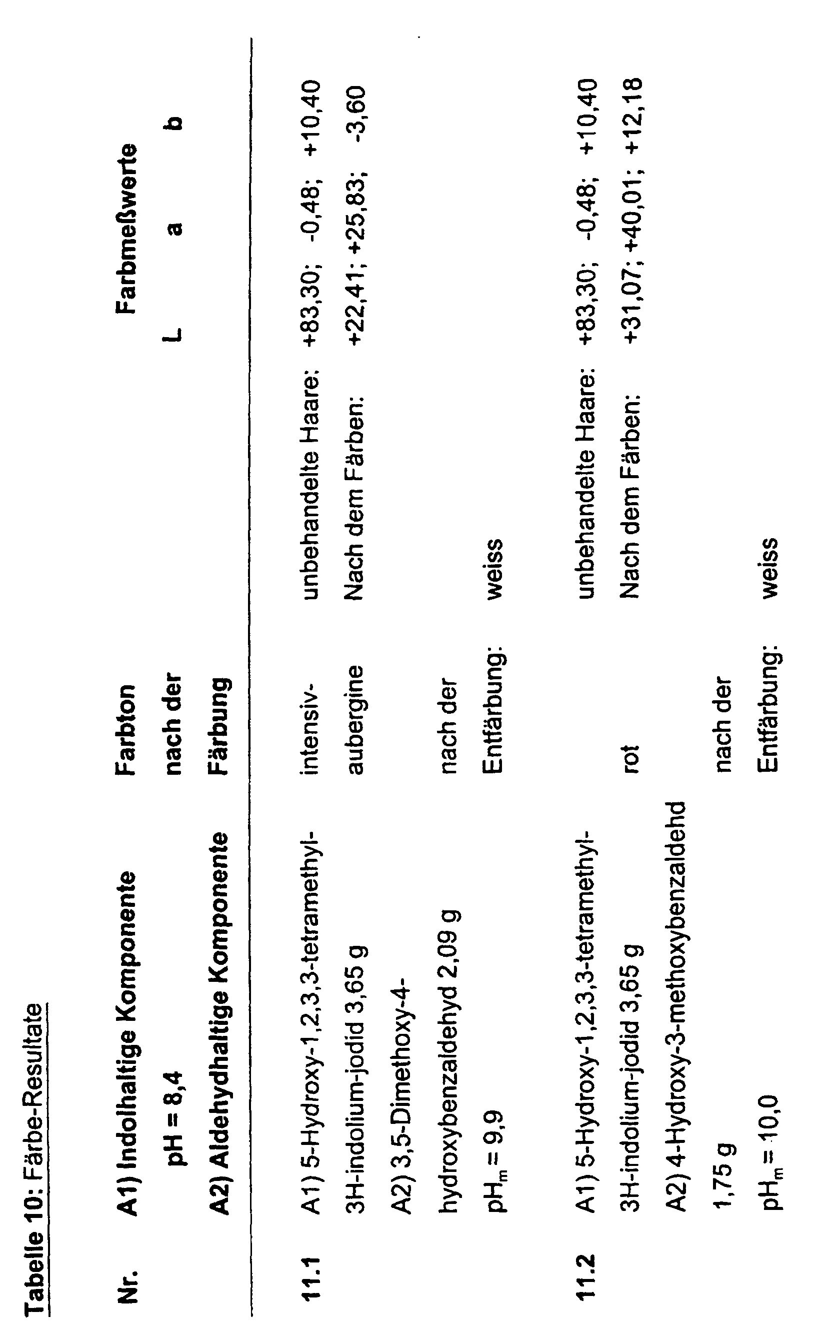

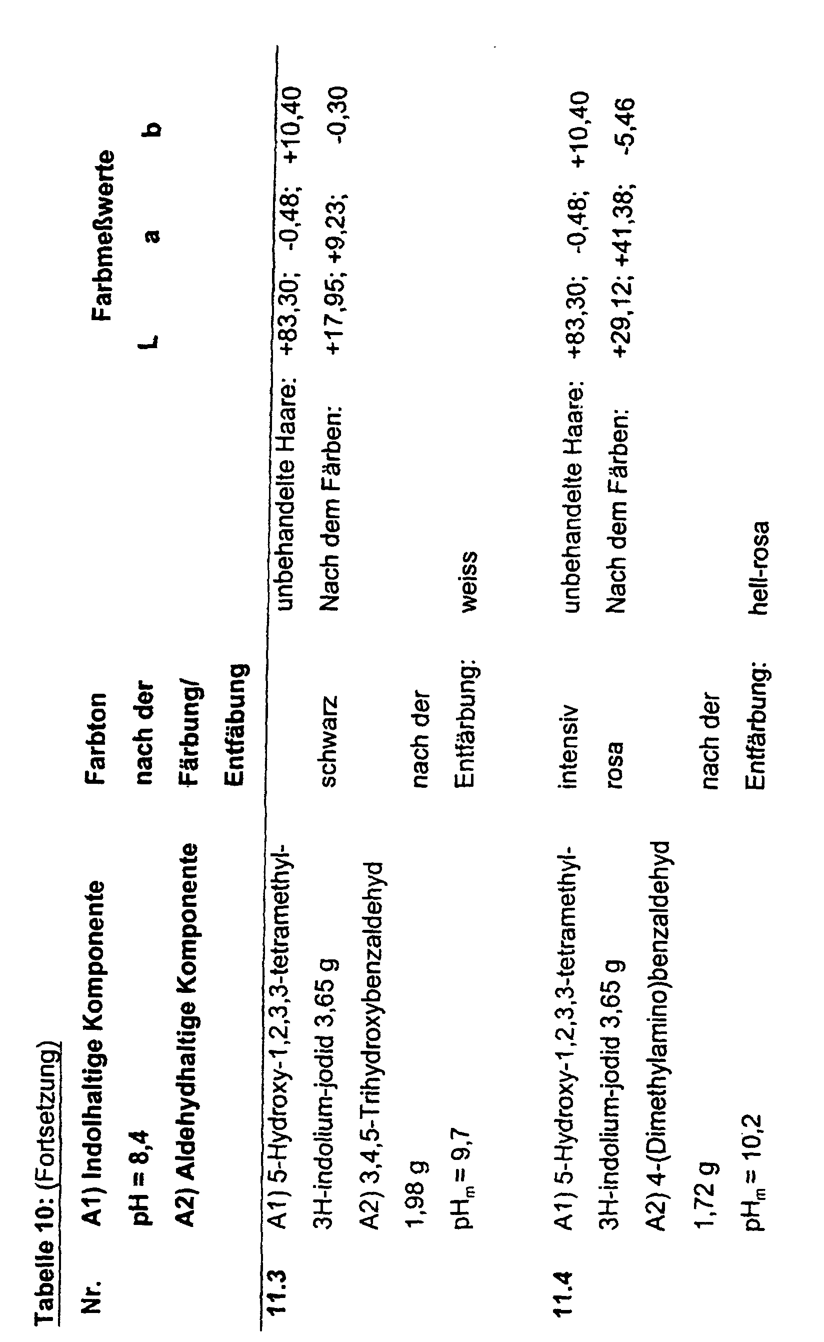

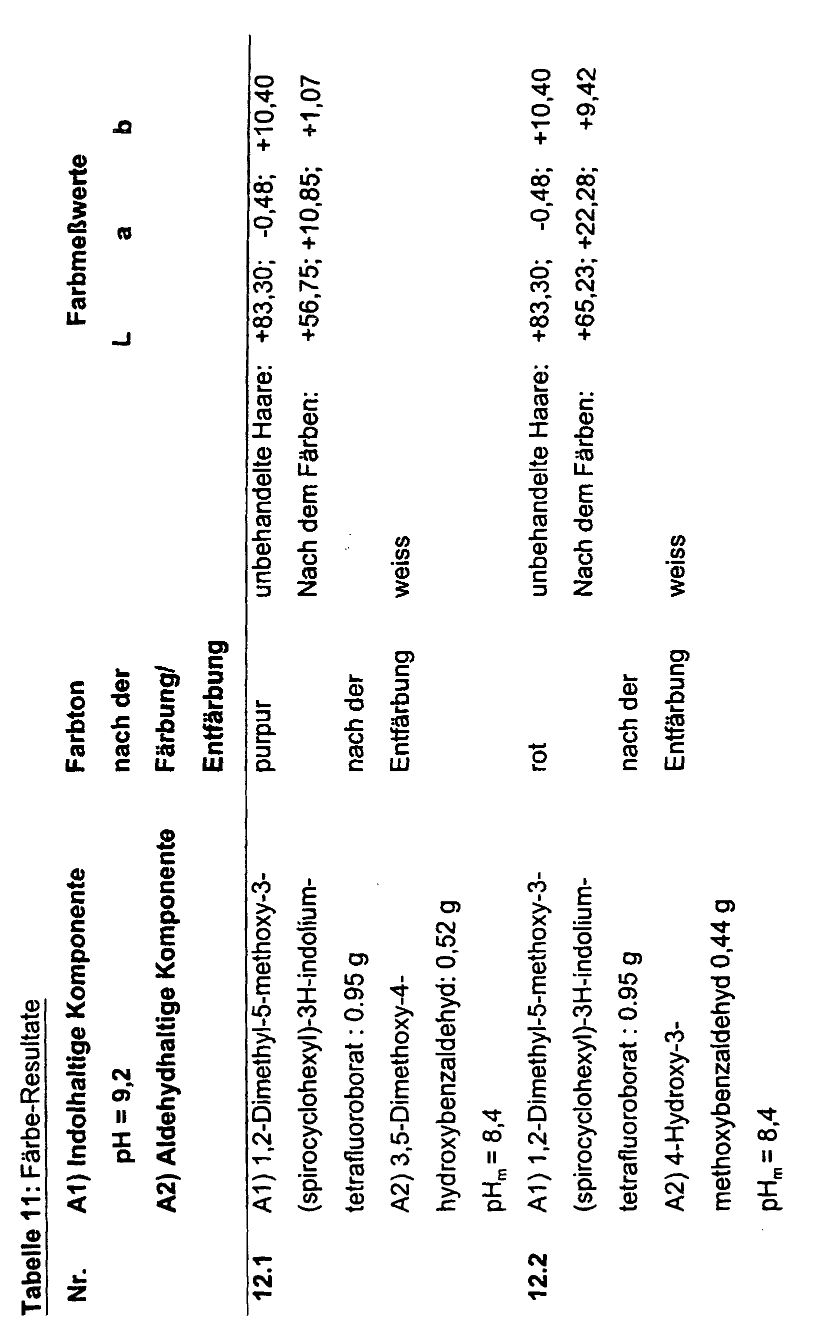

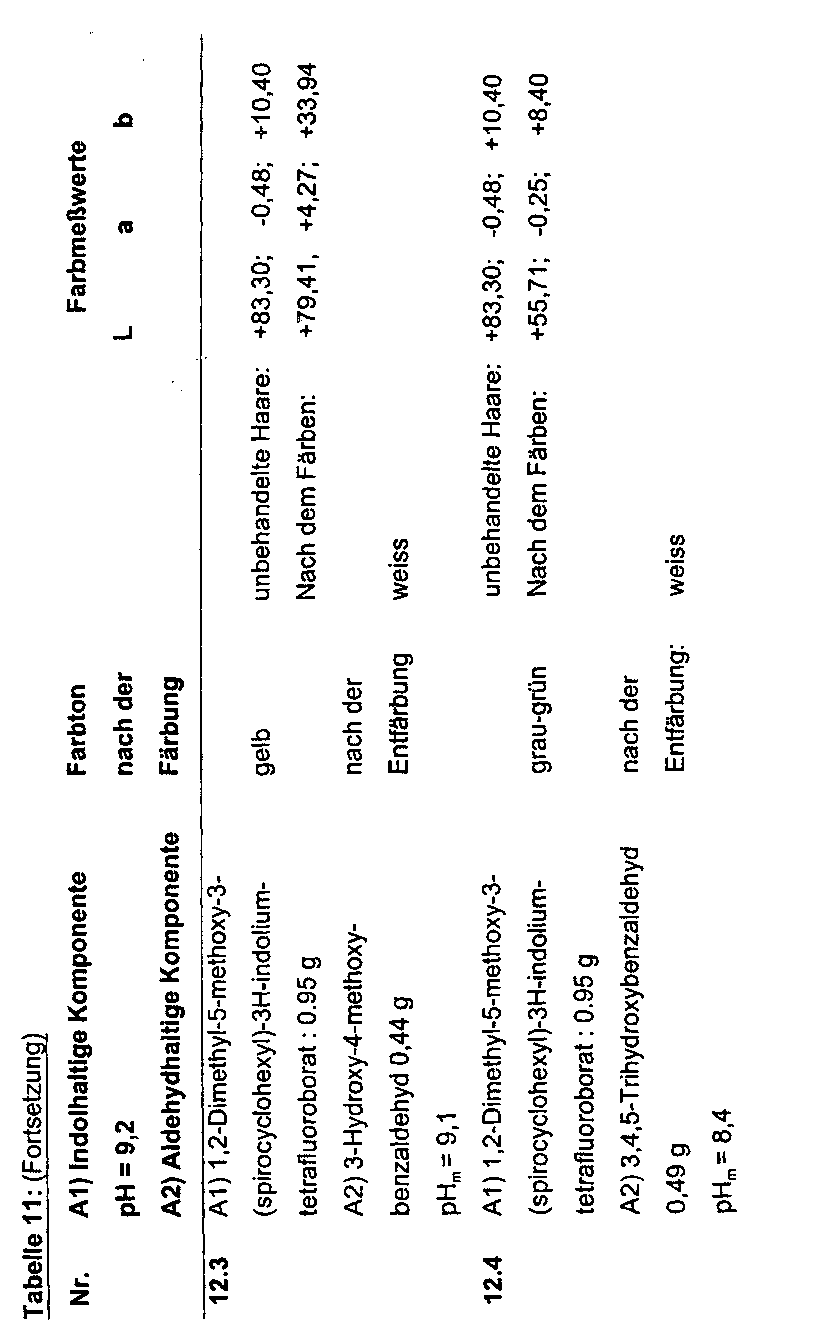

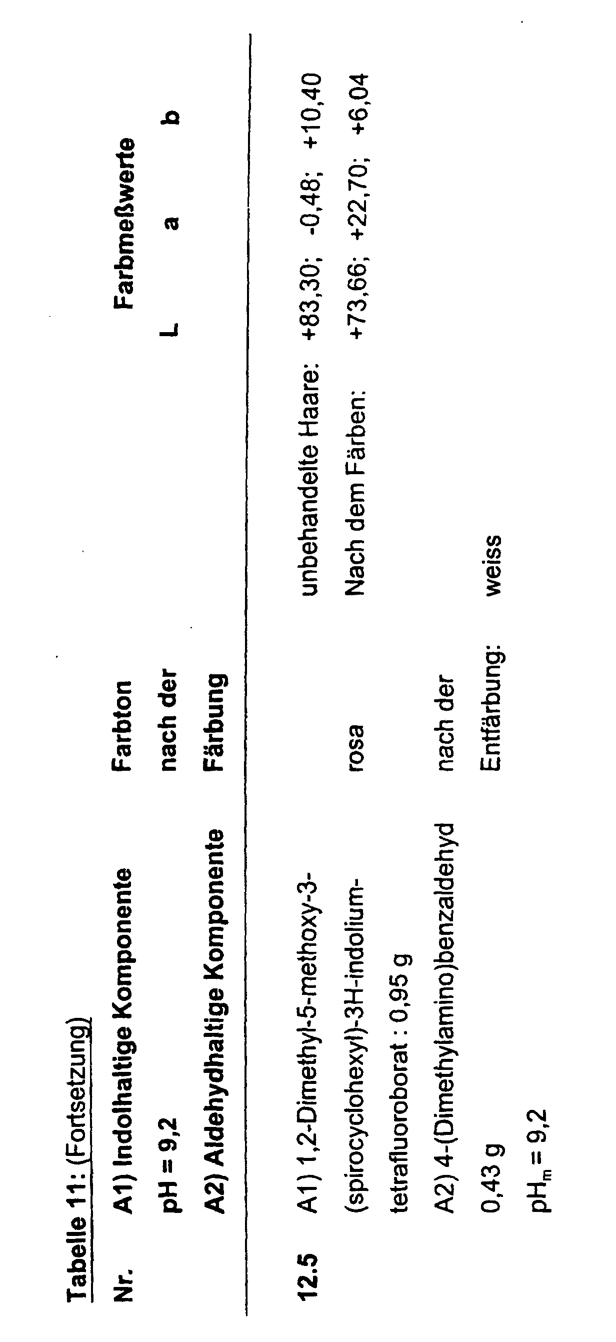

Die in den vorliegenden Tabellen 1 bis 10 angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

## Patentansprüche

1. Mittel zur Färbung von Fasern, welches durch Vermischen zweier Komponenten -falls erforderlich unter Zusatz eines Alkalisierungsmittels oder einer Säure- erhalten wird und **dadurch gekennzeichnet ist, dass** die eine Komponente (Komponente A2) mindestens eine Carbonylverbindung enthält, und die andere Komponente (Komponente A1) mindestens ein Indolinderivat der Formel (I) oder ein 3H-Indolium-Derivat der Formel (la) enthält, 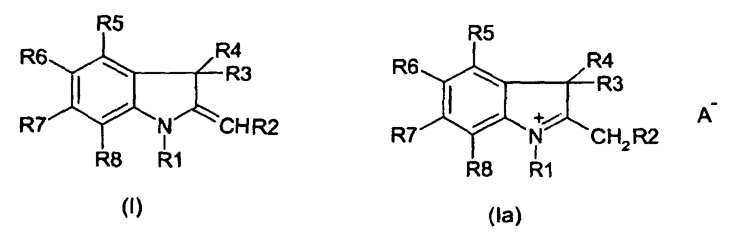 wobei in den Formeln (I) und (la) die Restgruppen R1 bis R8 und A⁻ die folgende Bedeutung haben:
R1 ist gleich einer geradkettigen oder verzweigten C1- bis C8-Alkylgruppe, C1- bis C8-Monohydroxyalkylgruppe, C2- bis C8-Polyhydroxyalkylgruppe, C1- bis C8-Alkoxy-(C1- bis C8)alkylgruppe oder Thio-(C1- bis C8)alkylgruppe, einer -(CH₂)ₙ-X-R^{a} -Gruppe, einer -(CH₂)ₘ-X-(CH₂)ₙ-Y-(CH₂)ₚ-R^{a} -Gruppe, einer -(CH₂)ₚ-R^{a} -Gruppe, einer -(CH₂)ₘ-Y-(CH₂)ₙ-X-(CH₂)_{P}-R^{a}-Gruppe, einer -(CH₂)ₘ-CO-(CH₂)ₚ-X-R^{a} - Gruppe, einer -(CH₂)ₘ-X-(CH₂)ₚ-CO-Y-R^{a} -Gruppe, oder gleich 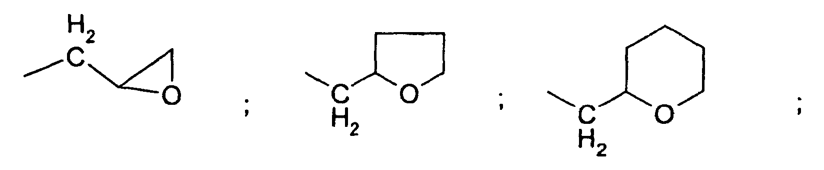 wobei X und Y unabhängig voneinander gleich einem Sauerstoffatom, einem Schwefelatom oder einer NR^{b}-Gruppe sind, R^{a} und R^{b} unabhängig voneinander gleich einem Wasserstoffatom, einem gegebenenfalls substituierten aromatischen Carbozyklus oder Heterocyclus oder einer geradkettigen oder verzweigten C1- bis C8-Alkylgruppe sind,
m und n unabhängig voneinander gleich einer ganzen Zahl von 1 bis 6 sind und p gleich einer ganzen Zahl von 0 bis 6 ist;
**R2** ist gleich einem Wasserstoffatom oder einer geradkettigen C1- bis C6-Alkylgruppe;
**R3** und **R4** sind unabhängig voneinander gleich einer geradkettigen oder verzweigten C1- bis C4-Alkylgruppe (insbesondere einer Methylgruppe), einer -(CH₂)ₙ-R^{c}, -(CH₂)ₘ-CHR^{c}-X-(CH₂)ₙ-R^{c}-Gruppe, einer -(CH₂)ₙ-CO-R^{c}-Gruppe , einer -(CH₂)ₙ-CO-XR^{c}-Gruppe, einer -(CH₂)ₙ-CN -Gruppe, einer. -(CH₂)ₙ-CH=C(CH₃)₂ -Gruppe, einer -(CH₂)ₘ-X-CHR^{c}-(CH₂)ₙ-R^{c} -Gruppe oder einer -(CH₂)ₙCH=CH -Gruppe ist, wobei X gleich einem Sauerstoffatom, einem Schwefelatom oder einer NR^{b}-Gruppe ist, m und n unabhängig voneinander gleich 1 bis 6 sind, und R^{c} für ein Wasserstoffatom, einen gegebenenfalls substituierten aromatischen Carbozyklus oder Heterocyclus oder eine geradkettige oder verzweigte C1- bis C6-Alkylgruppe steht; mit der Maßgabe, dass die Reste **R3** und **R4** auch gemeinsam, verbunden über eine (CH₂)ₙ-Gruppe (mit n=1-3), mit dem 3H-Kohlenstoff eine Spiroverbindung bilden können;
**R5, R6, R7** und **R8** sind unabhängig voneinander gleich einem Wasserstoffatom, einer geradkettigen oder verzweigten C1- bis C4-Alkylgruppe oder C1- bis C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethylgruppe, einer -CHO -Gruppe, einer -COR^{d}-Gruppe, einer -COOH -Gruppe, einer -CO₂R^{d} -Gruppe, einer -OCOR^{d} -Gruppe, einer -OCH₂Aryl -Gruppe, einer -SO₂NH₂ -Gruppe, einer -NH₂ -Gruppe, einer -NH₃⁺ -Gruppe, einer -NHR^{d} -Gruppe, einer -NH₂R^{d +} -Gruppe, einer -N(R^{d})₂-Gruppe, einer -N(R^{d})₃⁺ -Gruppe, einer -NHCOR^{d}-Gruppe, einer -NHCOOR^{d} -Gruppe, einer -CH₂NH₂ -Gruppe, einer -CH₂NHR^{d} -Gruppe, einer -CH₂N(R^{d})₂ -Gruppe, einer -CO₂CF₃ -Gruppe, einer -PO(OR^{d})₂-Gruppe, einer -SO₂CHF₂ -Gruppe, einer -SO₂CF₃ -Gruppe, einer -SO₂R^{d}-Gruppe oder einer -SR^{d} -Gruppe, wobei R^{d} gleich einem Wasserstoffatom, einem gegebenenfalls substituierten aromatischen Carbozyklus oder Heterocyclus oder einer C1- bis C6-Alkylgruppe ist, unter der Bedingung, dass (i) mindestens einer der Reste **R5** bis **R8** von Wasserstoff verschieden ist, und (ii) das Indolinderivat der Formel (I) nicht gleich 5-Chloro-2-methylen-1,3,3-trimethylindolin ist, wenn die Carbonylverbindung gleich 3,4-Dihydroxybenzaldehyd oder 4-Hydroxybenzaldehyd ist;
**A**^{**-**} ist gleich einem Anion einer organischen oder anorganischen Säure.

2. Mittel nach Anspruch 1, **dadurch gekennzeichnet, dass** die Verbindung der Formel (I)/(Ia) ausgewählt ist aus 1,3,3,4-Tetramethyl-2-methylen-indolin sowie dessen Salzen, 1,3,3,5-Tetramethyl-2-methylen-indolin sowie dessen Salzen, 1,3,3,6-Tetramethyl-2-methylen-indolin sowie dessen Salzen, 1,3,3,7-Tetramethyl-2-methylen-indolin sowie dessen Salzen, 1,3,3,6,7-Pentamethyl-2-methylen-indolin sowie dessen Salzen, 1,3,3,5,7-Pentamethyl-2-methylen-indolin sowie dessen Salzen, 1,3,3,4,7-Pentamethyl-2-methylen-indofin sowie dessen Salzen, 5-Fluoro-1,3,3-trimethyl-2-methylen-indolin sowie dessen Salzen, 5-Isopropyl-1,3,3-trimethyl-2-methylen-indolin sowie dessen Salzen, 5-Hydroxy-1,3,3-trimethyl-2-methylen-indolin sowie dessen Salzen, 5-Methoxy-1,3,3-trimethyl-2-methylen-indolin sowie dessen Salzen, 5-Nitro-1,3,3-trimethyl-2-methylen-indolin sowie dessen Salzen, 5-Amino-1,3,3-trimethyl-2-methylen-indolin sowie dessen Salzen, 5-N-acetylamino-1,3,3-trimethyl-2-methylen-indolin sowie dessen Salzen, 6-Hydroxy-1,3,3-trimethyl-2-methylen-indolin sowie dessen Salzen, 6-Methoxy-1,3,3-trimethyl-2-methylen-indolin sowie dessen Salzen, 5-Methoxy-6-nitro-1,3,3-trimethyl-2-methylen-indolin sowie dessen Salzen, 5-Methoxy-6-amino-1,3,3-trimethyl-2-methylen-indolin sowie dessen Salzen, 5-Methoxy-6-N-acetylamino-1,3,3-trimethyl-2-methylen-indolin sowie dessen Salzen, 5,6-Dihydroxy-1,3,3-trimethyl-2-methylen-indolin sowie dessen Salzen, 5,6-Dimethoxy-1,3,3-trimethyl-2-methylen-indolin sowie dessen Salzen, 5,6-Methylendioxy-1,3,3-trimethyl-2-methylenindolin sowie dessen Salzen, 4,5-Dihydroxy-1,3,3-trimethyl-2-methylenindolin sowie dessen Salzen, 5,7-Dihydroxy-1,3,3-trimethyl-2-methylenindolin sowie dessen Salzen, 5-Amino-6-methoxy-1,3,3-trimethyl-2-methylen-indolin sowie dessen Salzen, 5-Amino-7-hydroxy-1,3,3-trimethyl-2-methylen-indolin sowie dessen Salzen, 5-Hydroxy-7-amino-1,3,3-trimethyl-2-methylen-indolin sowie dessen Salzen, 5-Hydroxy-7-N-acetylamino-1,3,3-trimethyl-2-methylen-indolin sowie dessen Salzen, 1-Methyl-3-spirocyclohexyl-5-hydroxy-2-methylen-indolin sowie dessen Salzen und 1-Methyl-3-spirocyclohexyl-5-methoxy-2-rnethylen-indolin sowie dessen Salzen.

3. Mittel nach Anspruch 1 oder 2, **dadurch gekennzeichnet, dass** die Carbonylverbindung ausgewählt ist aus Vanillin, lsovanillin, 3,4-Dihydroxy-benzaldehyd, 4-Hydroxybenzaldehyd, 3,5-Dimethoxy-4-hydroxy-benzaldehyd, 4-Dimethylaminobenzaldehyd, 4-Methyl-5-imidazol-carboxaldehyd, 4- Dimethylamino-zimtaldehyd, 4-Hydroxy-2-methoxy-benzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxy-benzaldehyd, 3,4-Dihydroxy-benzaldehyd, 2,5-Dihydroxy-benzaldehyd, 2,3,4-Trihydroxybenzaldehyd, 3,4,5-Trihydroxy-benzaldehyd, 2,4,6-Trihydroxybenzaldehyd, 2,4-Dimethoxy-benzaldehyd, 2,3-Dimethoxy-benzaldehyd, 2,5-Dimethoxy-benzaldehyd, 3,5-Dimethoxybenzaldehyd, 3,4-Dimethoxy-benzaldehyd, Indol-3-carbaldehyd, Benzol-1,4-dicarb-aldehyd, 4-Ethoxybenzaldehyd, 2-Methyl-1,4-naphthochinon, 4-Carboxy-benzaldehyd, 4-Hydroxy-3-methoxy-zimtaldehyd, 3,5-Dimethoxy-4-hydroxy-zimtaldehyd, 3-Methoxy-4-(1-pyrrolidinyl)-benzaldehyd, 4-Diethylamino-3-methoxybenzaldehyd, 1,2-Phthaldialdehyd, Pyrrol-2-aldehyd, Thiophen-2-aldehyd, Thiophen-3-aldehyd, Chromone-3-carboxaldehyd, 6-Methyl-4-oxo-1(4H)-bebzopyran-3-carbaldehyd, N-Methylpyrrol-2-aldehyd, 5-Methylfurfural, 6-Hydroxychromen-3-carboxaldehyd, 6-Methylindol-3-carboxaldehyd, 4-Dibutylamino-benzaldehyd, N-Ethylcarbazol-3-aldehyd, 4-Diethylamino-2-hydroxy-benzaldehyd, 3,4-Dimethoxy-5-hydroxybenzaldehyd, 5-(4-(Diethyl-amino)phenyl)-2,4-pentadienal, 2,3-Thiophendicarboxaldehyd, 2,5-Thiophendicarboxaldehyd, 2-Methoxy-1-naphthatdehyd, 3-Ethoxy-4-hydroxybenzaldehyd, 2-Nitrobenzaldehyd, 3-Nitrobenzaldehyd und 4-Nitrobenzaldehyd.

4. Mittel nach einem der Ansprüche 1 bis 3, **dadurch gekennzeichnet, dass** die Verbindung der Formel (I) oder (Ia) in der Komponente A1 in einer Gesamtmenge von etwa 0,02 bis 20 Gewichtsprozent enthalten ist.

5. Mittel nach einem der Ansprüche 1 bis 4, **dadurch gekennzeichnet, dass** die Carbonylverbindung in der Komponente A2 in einer Gesamtmenge von etwa 0,02 bis 20 Gewichtsprozent enthalten ist.

6. Mittel nach einem der Ansprüche 1 bis 5, **dadurch gekennzeichnet, dass** die Verbindung der Formel (I) oder (Ia) und die Carbonylverbindung in dem durch Vermischen der Komponenten A1 und A2 erhaltenen gebrauchsfertigen Färbemittel jeweils in einer Gesamtmenge von etwa 0,01 bis 10 Gewichtsprozent enthalten ist.

7. Mittel nach einem der Ansprüche 1 bis 6, **dadurch gekennzeichnet, dass** es zusätzlich mindestens einen direktziehenden Farbstoff enthält.

8. Mittel nach einem der Ansprüche 1 bis 7, **dadurch gekennzeichnet, dass** der pH-Wert des gebrauchsfertigen Färbemittels gleich 3 bis 11 ist.

9. Mehrkomponenten-Kit zur Färbung und späteren Entfärbung von Fasern, **dadurch gekennzeichnet, dass** er ein Färbemittel (A) gemäss einem der Patentansprüche 1 bis 8 und eine mindestens ein Sulfit enthaltende, entfärbende Komponente (B) enthält.

10. Mehrkomponenten-Kit nach Anspruch 9, **dadurch gekennzeichnet, dass** das Entfärbemittel (B) einen pH-Wert von 3 bis 8 aufweist.

## Claims

1. Agent for dyeing fibres which is obtained by mixing two components - if necessary with the addition of an alkalizing agent or an acid - and is **characterized in that** the one component (component A2) comprises at least one carbonyl compound, and the other component (component A1) comprises at least one indoline derivative of the formula (I) or a 3H-indolium derivative of the formula (Ia), 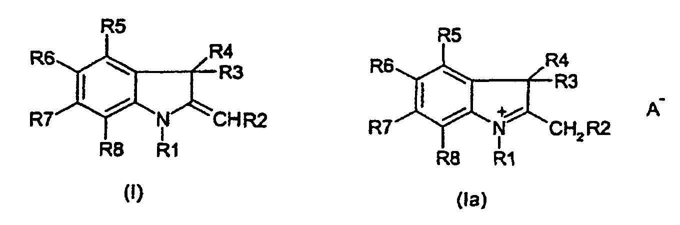 where in the formulae (I) and (Ia) the radical groups R1 to R8 and A⁻ have the following meanings:
**R1** is a straight-chain or branched C1- to C8-alkyl group, C1- to C8-monohydroxyalkyl group, C2- to C8-polyhydroxyalkyl group, C1- to C8-alkoxy(Cl- to C8)alkyl group or thio(C1- to C8)alkyl group, a -(CH₂)ₙ-X-R^{a} group, a -(CH₂)ₘ-X-(CH₂)ₙ-Y-(CH₂)ₚ-R^{a} group, a -(CH₂)ₚ-R^{a} group, a -(CH₂)ₘ-Y-(CH₂)ₙ-X-(CH₂)ₚ-R^{a} group, a -(CH₂)ₘ-CO-(CH₂)ₚ-X-R^{a} group, a -(CH₂)ₘ-X-(CH₂)ₚ-CO-Y-R^{a} group, or is 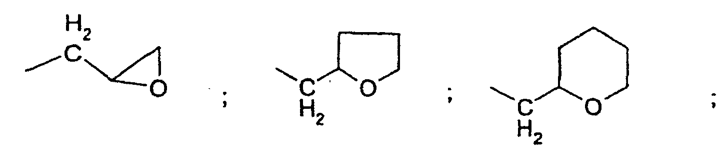 where X and Y, independently of one another, are an oxygen atom, a sulphur atom or an NR^{b} group, R^{a} and R^{b}, independently of one another, are a hydrogen atom, an optionally substituted aromatic carbocycle or heterocycle or a straight-chain or branched C1- to C8-alkyl group,
m and n, independently of one another, are an integer from 1 to 6 and p is an integer from 0 to 6;
**R2** is a hydrogen atom or a straight-chain C1- to C6-alkyl group;
**R3** and **R4,** independently of one another, are a straight-chain or branched C1- to C4-alkyl group (in particular a methyl group), a -(CH₂)ₙ-R^{c} group, a -(CH₂)ₘ-CHR^{c}-X-(CH₂)ₙ-R^{c} group, a -(CH₂)ₙ-CO-R^{c} group, a -(CH₂)ₘ-CO-XR^{c} group, a -(CH₂)ₙ-CN group, a -(CH₂)ₙ-CH=C(CH₃)₂ group, a - (CH₂)ₘ-X-CHR^{c}-(CH₂)ₙ-R^{c} group or a -(CH₂)ₙCH=CH group, where X is an oxygen atom, a sulphur atom or an NR^{b} group, m and n, independently of one another, are 1 to 6, and R^{c} is a hydrogen atom, an optionally substituted aromatic carbocycle or heterocycle or a straight-chain or branched C1- to C6-alkyl group; with the proviso that the radicals **R3** and **R4** may also together, bonded via a (CH₂)ₙ group (where n = 1-3), with the 3H carbon, form a spiro compound;
**R5, R6, R7** and **R8,** independently one another, are a hydrogen atom, a straight-chain or branched C1-to C4-alkyl group or C1- to C4-hydroxyalkyl group, a hydroxyl group, a methoxy group, a benzyl group, a halogen atom (F, Cl, Br, I), a nitro group, a nitroso group, a cyano group, a trifluoromethyl group, a -CHO group, a -COR^{d} group, a -COOH group, a -CO₂R^{d} group, an -OCOR^{d} group, an -OCH₂aryl group, an -SO₂NH₂ group, an -NH₂ group, an -NH₃⁺ group, an -NHR^{d} group, an -NH₂R^{d+} group, an -N(R^{d})₂ group, an -N(R^{d})₃⁺ group, an -NHCOR^{d} group, an -NHCOOR^{d} group, a -CH₂NH₂ group, a -CH₂NHR^{d} group, a -CH₂N(R^{d})₂ group, a -CO₂CF₃ group, a -PO(OR^{d})₂ group, an -SO₂CHF₂ group, an -SO₂CF₃ group, an -SO₂R^{d} group or an -SR^{d} group, where R^{d} is a hydrogen atom, an optionally substituted aromatic carbocycle or heterocycle or a C1- to C6-alkyl group, with the proviso that (i) at least one of the radicals **R5** to **R8** is different from hydrogen, and (ii) the indoline derivative of the formula (I) is not 5-chloro-2-methylene-1,3,3-trimethylindoline if the carbonyl compound is 3,4-dihydroxybenzaldehyde or 4-hydroxybenzaldehyde;
**A**^{**-**} is an anion of an organic or inorganic acid.

2. Agent according to Claim 1, **characterized in that** the compound of the formula (I)/(Ia) is chosen from
1,3,3,4-tetramethyl-2-methyleneindoline and salts thereof, 1,3,3,5-tetramethyl-2-methyleneindoline and salts thereof, 1,3,3,6-tetramethyl-2-methyleneindoline and salts thereof, 1,3,3,7-tetramethyl-2-methyleneindoline and salts thereof, 1,3,3,6,7-pentamethyl-2-methyleneindoline and salts thereof, 1,3,3,5,7-pentamethyl-2-methyleneindoline and salts thereof, 1,3,3,4,7-pentamethyl-2-methyleneindoline and salts thereof, 5-fluoro-1,3,3-trimethyl-2-methyleneindoline and salts thereof, 5-isopropyl-1,3,3-trimethyl-2-methyleneindoline and salts thereof, 5-hydroxy-1,3,3-trimethyl-2-methyleneindoline and salts thereof, 5-methoxy-1,3,3-trimethyl-2-methyleneindoline and salts thereof, 5-nitro-1,3,3-trimethyl-2-methyleneindoline and salts thereof, 5-amino-1,3,3-trimethyl-2-methyleneindoline and salts thereof, 5-N-acetylamino-1,3,3-trimethyl-2-methyleneindoline and salts thereof, 6-hydroxy-1,3,3-trimethyl-2-methyleneindoline and salts thereof, 6-methoxy-1,3,3-trimethyl-2-methyleneindoline and salts thereof, 5-methoxy-6-nitro-1,3,3-trimethyl-2-methyleneindoline and salts thereof, 5-methoxy-6-amino-1,3,3-trimethyl-2-methyleneindoline and salts thereof, 5-methoxy-6-N-acetylamino-1,3,3-trimethyl-2-methyleneindoline and salts thereof, 5,6-dihydroxy-1,3,3-trimethyl-2-methyleneindoline and salts thereof, 5,6-dimethoxy-1,3,3-trimethyl-2-methyleneindoline and salts thereof, 5,6-methylenedioxy-1,3,3-trimethyl-2-methyleneindoline and salts thereof, 4,5-dihydroxy-1,3,3-trimethyl-2-methyleneindoline and salts thereof, 5,7-dihydroxy-1,3,3-trimethyl-2-methyleneindoline and salts thereof, 5-amino-6-methoxy-1,3,3-trimethyl-2-methyleneindoline and salts thereof, 5-amino-7-hydroxy-1,3,3-trimethyl-2-methyleneindoline and salts thereof, 5-hydroxy-7-amino-1,3,3-trimethyl-2-methyleneindoline and salts thereof, 5-hydroxy-7-N-acetylamino-1,3,3-trimethyl-2-methyleneindoline and salts thereof, 1-methyl-3-spirocyclohexyl-5-hydroxy-2-methyleneindoline and salts thereof and 1-methyl-3-spirocyclohexyl-5-methoxy-2-methyleneindoline and salts thereof.

3. Agent according to Claim 1 or 2, **characterized in that** the carbonyl compound is chosen from vanillin, isovanillin, 3,4-dihydroxybenzaldehyde, 4-hydroxybenzaldehyde, 3,5-dimethoxy-4-hydroxybenzaldehyde, 4-dimethylaminobenzaldehyde, 4-methyl-5-imidazolecarboxaldehyde, 4-dimethyl-aminocinnamaldehyde, 4-hydroxy-2-methoxybenzaldehyde, 3,5-dimethyl-4-hydroxybenzaldehyde, 4-dimethylamino-2-methoxybenzaldehyde, 2-hydroxybenzaldehyde, 4-hydroxy-1-naphthaldehyde, 4-methoxy-1-naphthaldehyde, 4-dimethylamino-1-naphthaldehyde, 4'-hydroxybiphenyl-1-carbaldehyde, 2-hydroxy-3-methoxybenzaldehyde, 2,4-dihydroxybenzaldehyde, 3,4-dihydroxybenzaldehyde, 2,5-dihydroxybenzaldehyde, 2,3,4-trihydroxybenzaldehyde, 3,4,5-trihydroxybenzaldehyde, 2,4,6-trihydroxybenzaldehyde, 2,4-dimethoxybenzaldehyde, 2,3-dimethoxybenzaldehyde, 2,5-dimethoxybenzaldehyde, 3,5-dimethoxybenzaldehyde, 3,4-dimethoxybenzaldehyde, indole-3-carbaldehyde, benzene-1,4-dicarbaldehyde, 4-ethoxybenzaldehyde, 2-methyl-1,4-naphthoquinone, 4-carboxybenzaldehyde, 4-hydroxy-3-methoxycinnamaldehyde, 3,5-dimethoxy-4-hydroxycinnamaldehyde, 3-methoxy-4-(1-pyrrolidinyl)benzaldehyde, 4-diethylamino-3-methoxybenzaldehyde, 1,2-phthaldialdehyde, pyrrole-2-aldehyde, thiophene-2-aldehyde, thiophene-3-aldehyde, chromone-3-carboxaldehyde, 6-methyl-4-oxo-1(4H)-benzopyran-3-carbaldehyde, N-methylpyrrole-2-aldehyde, 5-methylfurfural, 6-hydroxychromene-3-carboxaldehyde, 6-methylindole-3-carboxaldehyde, 4-dibutylaminobenzaldehyde, N-ethylcarbazole-3-aldehyde, 4-diethylamino-2-hydroxybenzaldehyde, 3,4-dimethoxy-5-hydroxybenzaldehyde, 5-(4-(diethylamino)phenyl)-2,4-pentadienal, 2,3-thiophenedicarboxaldehyde, 2,5-thiophenedicarboxaldehyde, 2-methoxy-1-naphthaldehyde, 3-ethoxy-4-hydroxybenzaldehyde, 2-nitrobenzaldehyde, 3-nitrobenzaldehyde and 4-nitrobenzaldehyde.

4. Agent according to one of Claims 1 to 3, **characterized in that** the compound of the formula (I) or (Ia) is present in component A1 in a total amount of from about 0.02 to 20 per cent by weight.

5. Agent according to one of Claims 1 to 4, **characterized in that** the carbonyl compound is present in component A2 in a total amount of from about 0.02 to 20 per cent by weight.

6. Agent according to one of Claims 1 to 5, **characterized in that** the compound of the formula (I) or (Ia) and the carbonyl compound in the ready-to-use dyeing agent obtained by mixing components A1 and A2 is in each case present in a total amount of from about 0.01 to 10 per cent by weight.

7. Agent according to one of Claims 1 to 6, **characterized in that** it additionally comprises at least one direct dye.

8. Agent according to one of Claims 1 to 7, **characterized in that** the pH of the ready-to-use dyeing agent is 3 to 11.

9. Multicomponent kit for the dyeing and subsequent decolouring of fibres, **characterized in that** it comprises a dyeing agent (A) according to one of Patent Claims 1 to 8 and a decolouring component (B) comprising at least one sulphite.

10. Multicomponent kit according to Claim 9, **characterized in that** the decolouring agent (B) has a pH of from 3 to 8.

## Revendications

1. Composition pour la teinture de fibres, qui est obtenue par mélange de deux composants - si nécessaire avec addition d'un agent d'alcalinisation ou d'un acide - et est **caractérisée en ce qu'**un composant (composant A2) contient au moins un composé carbonyle et l'autre composant (composant A1) contient au moins un dérivé d'indoline de formule (I) ou un dérivé de 3H-indolium de formule (Ia), 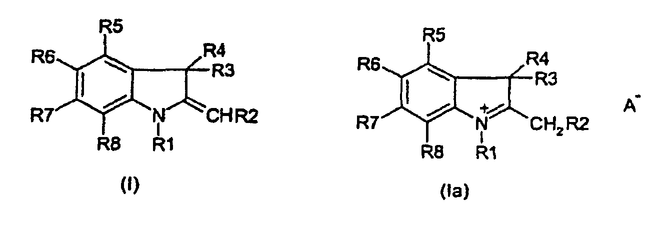 les radicaux R1 à R8 et A⁻ dans les formules (I) et (Ia) ayant les significations suivantes :
**R1** représente un groupe alkyle en C₁-C₈ à chaîne droite ou ramifiée, un groupe monohydroxyalkyle en C₁-C₈, un groupe polyhydroxyalkyle en C₂-C₈, un groupe alcoxy(C₁-C₈)-alkyle(C₁-C₈) ou un groupe thioalkyle en C₁-C₈, un groupe -(CH₂)ₙ-X-R^{a}, un groupe -(CH₂)ₘ-X-(CH₂)ₙ-Y-(CH₂)ₚ-R^{a}, un groupe -(CH₂)ₚ-R^{a}, un groupe -(CH₂)ₘ-Y-(CH₂)ₙ-X-(CH₂)ₚ-R^{a}, un groupe -(CH₂)ₘ-CO-(CH₂)ₚ-X-R^{a}, un groupe -(CH₂)ₘ-X-(CH₂)ₚ-CO-Y-R^{a} ou 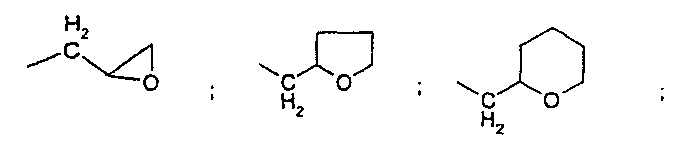
X et Y représentant, indépendamment l'un de l'autre, un atome d'oxygène, un atome de soufre ou un groupe NR^{b}, R^{a} et R^{b} représentant, indépendamment l'un de l'autre, un atome d'hydrogène, un cycle carbocyclique ou hétérocyclique aromatique éventuellement substitué ou un groupe alkyle en C₁-C₈ à chaîne droite ou ramifiée ;
m et n représentant, indépendamment l'un de l'autre, un nombre entier allant de 1 à 6, et p représentant un nombre entier allant de 0 à 6 ;
**R2** représente un atome d'hydrogène ou un groupe alkyle en C₁-C₆ à chaîne droite ;
**R3** et **R4** représentent, indépendamment l'un de l'autre, un groupe alkyle en C₁-C₄ à chaîne droite ou ramifiée (en particulier un groupe méthyle), un groupe - (CH₂)ₙ-R^{c}, un groupe -(CH₂)ₘ-CHR^{c}-X-(CH₂)ₙ-R^{c}, un groupe -(CH₂)ₙ-CO-R^{c}, un groupe -(CH₂)ₙ-CO-XR^{c}, un groupe -(CH₂)ₙ-CN, un groupe - (CH₂)ₙ-CH=C (CH₃)₂, un groupe -(CH₂)ₘ-X-CHR^{c}-(CH₂)ₙ-R^{c} ou un groupe -(CH₂)ₙCH=CH, X représentant un atome d'oxygène, un atome de soufre ou un groupe NR^{b}, m et n représentant, indépendamment l'un de l'autre, 1 à 6, et R^{c} représentant un atome d'hydrogène, un cycle carbocyclique ou hétérocyclique aromatique éventuellement substitué ou un groupe alkyle en C₁-C₆ à chaîne droite ou ramifiée ; étant entendu que les radicaux **R3** et **R4** peuvent également former ensemble, reliés par un groupe (CH₂)ₙ (où n = 1-3), avec l'atome de carbone 3H, un groupe spiro ;
**R5, R6, R7** et **R8** représentent, indépendamment les uns des autres, un atome d'hydrogène, un groupe alkyle en C₁-C₄ ou hydroxyalkyle en C₁-C₄, à chaîne droite ou ramifiée, un groupe hydroxy, un groupe méthoxy, un groupe benzyle, un atome d'halogène (F, Cl, Br, I), un groupe nitro, un groupe nitroso, un groupe cyano, un groupe trifluorométhyle, un groupe -CHO, un groupe -COR^{d}, un groupe -COOH, un groupe -CO₂R^{d}, un groupe -OCOR^{d}, un groupe -OCH₂-aryle, un groupe -SO₂NH₂, un groupe -NH₂, un groupe -NH₃⁺, un groupe -NHR^{d}, un groupe -NH₂R^{d+}, un groupe -N(R^{d})₂, un groupe -N(R^{d})₃⁺, un groupe -NHCOR^{d}, un groupe -NHCOOR^{d}, un groupe -CH₂NH₂, un groupe -CH₂NHR^{d}, un groupe -CH₂N(R^{d})₂, un groupe -CO₂CF₃, un groupe -PO(OR^{d})₂, un groupe -SO₂CHF₂, un groupe -SO₂CF₃, un groupe -SO₂R^{d} ou un groupe -SR^{d}, R^{d} représentant un atome d'hydrogène, un cycle carbocyclique ou hétérocyclique aromatique éventuellement substitué ou un groupe alkyle en C₁-C₆, étant entendu que (i) au moins l'un des radicaux **R5** à **R8** est différent d'un atome d'hydrogène et (ii) le dérivé d'indoline de formule (I) n'est pas la 5-chloro-2-méthylène-1,3,3-triméthylindoline lorsque le composé carbonyle est le 3,4-dihydroxybenzaldéhyde ou le 4-hydroxybenzaldéhyde ;
**A**^{**-**} représente un anion d'un acide organique ou minéral.

2. Composition selon la revendication 1, **caractérisée en ce que** le composé de formule (I)/(Ia) est choisi parmi
la 1,3,3,4-tétraméthyl-2-méthylène-indoline ainsi que ses sels, la 1,3,3,5-tétraméthyl-2-méthylène-indoline ainsi que ses sels, la 1,3,3,6-tétraméthyl-2-méthylène-indoline ainsi que ses sels, la 1,3,3,7-tétraméthyl-2-méthylène-indoline ainsi que ses sels, la 1,3,3,6,7-pentaméthyl-2-méthylène-indoline ainsi que ses sels, la 1,3,3,5,7-pentaméthyl-2-méthylène-indoline ainsi que ses sels, la 1,3,3,4,7-pentaméthyl-2-méthylène-indoline ainsi que ses sels, la 5-fluoro-1,3,3-triméthyl-2-méthylène-indoline ainsi que ses sels, la 5-isopropyl-1,3,3-triméthyl-2-méthylène-indoline ainsi que ses sels, la 5-hydroxy-1,3,3-triméthyl-2-méthylène-indoline ainsi que ses sels, la 5-méthoxy-1,3,3-triméthyl-2-méthylène-indoline ainsi que ses sels, la 5-nitro-1,3,3-triméthyl-2-méthylène-indoline ainsi que ses sels, la 5-amino-1,3,3-triméthyl-2-méthylène-indoline ainsi que ses sels, la 5-N-acétylamino-1,3,3-triméthyl-2-méthylène-indoline ainsi que ses sels, la 6-hydroxy-1,3,3-triméthyl-2-méthylène-indoline ainsi que ses sels, la 6-méthoxy-1,3,3-triméthyl-2-méthylène-indoline ainsi que ses sels, la 5-méthoxy-6-nitro-1,3,3-triméthyl-2-méthylène-indoline ainsi que ses sels, la 5-méthoxy-6-amino-1,3,3-triméthyl-2-méthylène-indoline ainsi que ses sels, la 5-méthoxy-6-N-acétylamino-1,3,3-triméthyl-2-méthylène-indoline ainsi que ses sels, la 5,6-dihydroxy-1,3,3-triméthyl-2-méthylène-indoline ainsi que ses sels, la 5,6-diméthoxy-1,3,3-triméthyl-2-méthylène-indoline ainsi que ses sels, la 5,6-méthylènedioxy-1,3,3-triméthyl-2-méthylène-indoline ainsi que ses sels, la 4,5-dihydroxy-1,3,3-triméthyl-2-méthylène-indoline ainsi que ses sels, la 5,7-dihydroxy-1,3,3-triméthyl-2-méthylène-indoline ainsi que ses sels, la 5-amino-6-méthoxy-1,3,3-triméthyl-2-méthylène-indoline ainsi que ses sels, la 5-amino-7-hydroxy-1,3,3-triméthyl-2-méthylène-indoline ainsi que ses sels, la 5-hydroxy-7-amino-1,3,3-triméthyl-2-méthylène-indoline ainsi que ses sels, la 5-hydroxy-7-N-acétylamino-1,3,3-triméthyl-2-méthylène-indoline ainsi que ses sels, la 1-méthyl-3-spirocyclohexyl-5-hydroxy-2-méthylène-indoline ainsi que ses sels et la 1-méthyl-3-spirocyclohexyl-5-méthoxy-2-méthylène-indoline ainsi que ses sels.

3. Composition selon la revendication 1 ou 2,
**caractérisée en ce que** le composé carbonyle est choisi parmi la vanilline, l'isovanilline, le 3,4-dihydroxybenzaldéhyde, le 4-hydroxybenzaldéhyde, le 3,5-diméthoxy-4-hydroxybenzaldéhyde, le 4-diméthylaminobenzaldéhyde, le 4-méthyl-5-imidazole-carboxaldéhyde, le 4-diméthylaminocinnamaldéhyde, le 4-hydroxy-2-méthoxybenzaldéhyde, le 3,5-diméthyl-4-hydroxybenzaldéhyde, le 4-diméthylamino-2-méthoxybenzaldéhyde, le 2-hydroxybenzaldéhyde, le 4-hydroxy-1-naphtaldéhyde, le 4-méthoxy-1-naphtaldéhyde, le 4-diméthylamino-1-naphtaldéhyde, le 4'-hydroxybiphényl-1-carbaldéhyde, le 2-hydroxy-3-méthoxybenzaldéhyde, le 2,4-dihydroxybenzaldéhyde, le 3,4-dihydroxybenzaldéhyde, le 2,5-dihydroxybenzaldéhyde, le 2,3,4-trihydroxybenzaldéhyde, le 3,4,5-trihydroxybenzaldéhyde, le 2,4,6-trihydroxybenzaldéhyde, le 2,4-diméthoxybenzaldéhyde, le 2,3-diméthoxybenzaldéhyde, le 2,5-diméthoxybenzaldéhyde, le 3,5-diméthoxybenzaldéhyde, le 3,4-diméthoxybenzaldéhyde, l'indole-3-carbaldéhyde, le benzène-1,4-dicarbaldéhyde, le 4-éthoxybenzaldéhyde, la 2-méthyl-1,4-naphtoquinone, le 4-carboxybenzaldéhyde, le 4-hydroxy-3-méthoxycinnamaldéhyde, le 3,5-diméthoxy-4-hydroxycinnamaldéhyde, le 3-méthoxy-4-(1-pyrrolidinyl)benzaldéhyde, le 4-diéthylamino-3-méthoxybenzaldéhyde, le 1,2-phtaldialdéhyde, le pyrrole-2-aldéhyde, le thiophène-2-aldéhyde, le thiophène-3-aldéhyde, le chromone-3-carboxaldéhyde, le 6-méthyl-4-oxo-1(4H)-benzopyranne-3-carbaldéhyde, le N-méthylpyrrol-2-aldéhyde, le 5-méthylfurfural, le 6-hydroxychromène-3-carboxaldéhyde, le 6-méthylindole-3-carboxaldéhyde, le 4-dibutylaminobenzaldéhyde, le N-éthylcarbazole-3-aldéhyde, le 4-diéthylamino-2-hydroxybenzaldéhyde, le 3,4-diméthoxy-5-hydroxybenzaldéhyde, le 5-(4-(diéthylamino)phényl)-2,4-pentadiénal, le 2,3-thiophènedicarboxaldéhyde, le 2,5-thiophènedicarboxaldéhyde, le 2-méthoxy-1-naphtaldéhyde, le 3-éthoxy-4-hydroxybenzaldéhyde, le 2-nitrobenzaldéhyde, le 3-nitrobenzaldéhyde et le 4-nitrobenzaldéhyde.

4. Composition selon l'une quelconque des revendications 1 à 3, **caractérisée en ce que** le composé de formule (I) ou (Ia) dans le composant A1 est contenu en une quantité totale d'environ 0,02 à 20 % en poids.

5. Composition selon l'une quelconque des revendications 1 à 4, **caractérisée en ce que** le composé carbonyle dans le composant A2 est contenu en une quantité totale d'environ 0,02 à 20 % en poids.

6. Composition selon l'une quelconque des revendications 1 à 5, **caractérisée en ce que** le composé de formule (I) ou (Ia) et le composé carbonyle dans la composition de teinture prête à l'emploi, obtenue par mélange des composants A1 et A2, sont contenus chacun en une quantité totale d'environ 0,01 à 10 % en poids.

7. Composition selon l'une quelconque des revendications 1 à 6, **caractérisée en ce qu'**elle contient en outre au moins un colorant direct.

8. Composition selon l'une quelconque des revendications 1 à 7, **caractérisée en ce que** le pH de la composition de teinture prête à l'emploi est dans l'intervalle allant de 3 à 11.

9. Nécessaire à plusieurs composants pour la coloration et la décoloration ultérieure de fibres, **caractérisé en ce qu'**il contient une composition de teinture (A) selon l'une quelconque des revendications 1 à 8 et un composant décolorant (B) contenant au moins un sulfite.

10. Nécessaire à plusieurs composants selon la revendication 9, **caractérisé en ce que** le composant décolorant (B) présente un pH de 3 à 8.
